(19) Europäisches Patentamt
European Patent Office
Office européen des brevets

(11) EP 1 096 926 B1

(12) EUROPEAN PATENT SPECIFICATION

(45) Date of publication and mention of the grant of the patent:
**28.02.2007 Bulletin 2007/09**

(21) Application number: **99933987.2**

(22) Date of filing: **12.07.1999**

(51) Int Cl.:
***A61K 31/137*** *(2006.01)* ***A61P 25/24*** *(2006.01)*

(86) International application number:
**PCT/US1999/015857**

(87) International publication number:
**WO 2000/002551 (20.01.2000 Gazette 2000/03)**

(54) **METHODS AND COMPOUNDS FOR TREATING DEPRESSION**

VERFAHREN UND VERBINDUNGEN ZUR BEHANDLUNG DER DEPRESSION

PROCEDES ET COMPOSES PERMETTANT DE TRAITER LA DEPRESSION

(84) Designated Contracting States:
**AT BE CH CY DE DK ES FI FR GB GR IE IT LI LU MC NL PT SE**

(30) Priority: **13.07.1998 US 92546 P**

(43) Date of publication of application:
**09.05.2001 Bulletin 2001/19**

(73) Proprietor: **NPS PHARMACEUTICALS, INC.**
**Salt Lake City, UT 84108 (US)**

(72) Inventors:
- **MUELLER, Alan**
  **Salt Lake City, UT 84108 (US)**
- **MOE, Scott**
  **Salt Lake City, UT 84121 (US)**
- **BALANDRIN, Manuel**
  **Sandy, UT 84093 (US)**

(74) Representative: **Vossius & Partner**
**Siebertstrasse 4**
**81675 München (DE)**

(56) References cited:

| | |
|---|---|
| EP-A1- 0 000 322 | WO-A1-95/15959 |
| WO-A1-96/40097 | WO-A1-97/46511 |
| WO-A1-98/56752 | WO-A2-95/21612 |
| FR-A5- 2 077 916 | GB-A- 1 129 029 |
| GB-A- 1 129 210 | GB-A- 1 363 986 |
| US-A- 3 258 488 | US-A- 3 407 258 |
| US-A- 3 449 427 | US-A- 4 003 932 |
| US-A- 4 018 895 | US-A- 4 313 896 |
| US-A- 4 314 081 | |

- **WILKINSON A ET AL: "Alaproclate acts as a potent, reversible and noncompetitive antagonist of the NMDA receptor coupled ion flow." JOURNAL OF PHARMACOLOGY AND EXPERIMENTAL THERAPEUTICS, (1994 DEC) 271 (3) 1314-9., XP000862851**
- **MARYANOFF ET AL: "Structure-Activity Studies on Antidepresant 2,2-Diarylethylamines" JOURNAL OF MEDICINAL CHEMISTRY,US, AMERICAN CHEMICAL SOCIETY. WASHINGTON, vol. 27, page 1067-1071 XP002083434 ISSN: 0022-2623**
- **CARNMALM, BERNT ET AL: "Antidepressant agents. V. 1,1-Dimethyl-.omega.,.omega.-diphenylalkylamines" ACTA PHARM. SUEC. (1975), 12(2), 173-82, XP000862860**
- **MARCUSSON J O ET AL: "INHIBITION OF U3HPAROXETINE BINDING BY VARIOUS SEROTONIN UPTAKE INHIBITORS: STRUCTURE-ACTIVITY RELATIONSHIPS" EUROPEAN JOURNAL OF PHARMACOLOGY, NL,AMSTERDAM, vol. 215, no. 2/03, page 191-198 XP002016630 ISSN: 0014-2999**
- **NOWAK G ET AL: "Adaptation of cortical NMDA receptors by chronic treatment with specific serotonin reuptake inhibitors." EUROPEAN JOURNAL OF PHARMACOLOGY, (1998 JAN 26) 342 (2-3) 367-70., XP000862854**
- **PAUL I A ET AL: "ADAPTATION OF THE N-METHYL-D-ASPARTATE RECEPTOR COMPLEX FOLLOWING CHRONIC ANTIDEPRESSANT TREATMENTS" JOURNAL OF PHARMACOLOGY AND EXPERIMENTAL THERAPEUTICS,US, AMERICAN SOCIETY FOR PHARMACOLOGY AND, vol. 269, no. 1, page 95-102 XP002016627 ISSN: 0022-3565**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

(Cont. next page)

- **BUCK K J ET AL: "STRUCTURAL DOMAINS OF CATECHOLAMINE TRANSPORTER CHIMERAS INVOLVED IN SELECTIVE INHIBITION BY ANTIDEPRESSANTS AND PSYCHOMOTOR STIMULANTS" MOLECULAR PHARMACOLOGY, BALTIMORE, MD, US, vol. 48, no. 6, December 1995 (1995-12), pages 1030-1037, XP000862850 ISSN: 0026-895X**
- **ANDREWS P R ET AL: "A PHARMACOPHORE HYPOTHESIS FOR ANTIDEPRESSANT ACTIVITY" QUANTITATIVE STRUCTURE-ACTIVITY RELATIONSHIPS, VCH PUBLISHERS, DEERFIELD BEACH, FL, US, vol. 6, no. 3, September 1997 (1997-09), pages 97-103, XP000862864 ISSN: 0931-8771**
- **MELLONI P ET AL: "POTENTIAL ANTIDEPRESSANT AGENTS-ALPHA-ARYLOXY-BENZYL DERIVATIVES OF ETHANOLAMINE AND MORPHOLINE" EUROPEAN JOURNAL OF MEDICINAL CHEMISTRY, EDITIONS SCIENTIFIQUE ELSEVIER, PARIS, FR, vol. 19, no. 3, 1984, pages 235-242, XP000605241 ISSN: 0223-5234**
- **"THE MERCK INDEX" 1996 , MERCK & CO.,INC XP002248270 * page 1621, Tofenacin * * page 709, Fluoxetine ***
- **ZEE VAN DER P ET AL: "A COMPARISON OF THE INHIBITORY EFFECTS OF AROMATIC SUBSTITUTED BENZHYDRYL ETHERS ON THE UPTAKE OF CATECHOLAMINES AND SEROTONIN INTO SYNAPTOSOMAL PREPARATIONS OF THE RAT BRAIN" NEUROPHARMACOLOGY, PERGAMON PRESS, OXFORD, GB, vol. 17, no. 7, July 1978 (1978-07), pages 483-490, XP000862833 ISSN: 0028-3908**
- **ZEE VAN DER P ET AL: "INFLUENCE OF ORPHENADRINE HCI AND ITS N-DEMETHYLATED DERIVATIVES ON THE INVITRO UPTAKE OF NORADRENALINE AND 5-HYDROXYTRYPTAMINE BY RAT BRAIN SLICES" NEUROPHARMACOLOGY, PERGAMON PRESS, OXFORD, GB, vol. 12, no. 9, 1973, pages 843-851, XP000862863 ISSN: 0028-3908**
- **DATABASE CA [Online] CHEMICAL ABSTRACTS SERVICE, COLUMBUS, OHIO, US; DAVIS, MARTIN A.: "Antidepressant dibenzo[a,d] cycloheptene derivatives" retrieved from STN Database accession no. 76:112871 XP002248271 & CA 890 423 A (AYERST, MCKENNA AND HARRISON LTD.) 11 January 1972**

## Description

This application claims priority to provisional application serial number 60/092,546.

This invention relates to the use of compounds for the preparation of a medicament for the treatment of depression. More specifically, the compounds are active at monoamine reuptake sites, such as the serotonin reuptake site; and the NMDA receptor.

The following description provides a summary of information relevant to the present invention. It is not an admission that any of the information provided herein is prior art to the presently claimed invention, nor that any of the publications specifically or implicitly referenced are prior art to the present invention.

Depression is a common illness associated with substantial morbidity and mortality. Major depression is characterized by feelings of despair, intense sadness, mental slowing, loss of concentration, pessimistic worry, agitation, and self-deprecation. Physical changes which can accompany depression, particularly in severe or "melancholic" depression, include insomnia or hypersomnia; anorexia and weight loss (or occasionally overeating); less energy and libido; and disruption of normal circadian rhythms of activity, body temperature, and different endocrine functions. (Baldessarini, in Goodman & Gilman's THE PHARMACOLOGICAL BASIS OF THERAPEUTICS, 9th Ed. Chapt. 19; McGraw-Hill, 1996.).

It is well known that compounds which block the reuptake of monoamines such as serotonin (serotonin-selective reuptake site inhibitors or SSRIs) possess antidepressant activity (U.S. Pat. Nos. 4,314,081, and U.S. Pat. No. 4,626,549, Molley and-Schmiegel). Also, several lines of evidence suggest that NMDA receptor antagonists which demonstrate functional antidepressant activity in several animal models (Skolnick, P., Editor, "Antidepressant: New Pharmacological Strategies", National Institutes of Health, Bethesda MD, 1998), may provide a useful approach to treating depression.

However, prior to the present invention, it was not recognized that compounds which are active at both monoamine reuptake sites, including the serotonin reuptake site, and the N-methyl-D-aspartate (NMDA) receptor and the use of such multi-active compounds would be beneficial for treating depression and other disorders.

The present invention features compounds active at both the serotonin reuptake site and the N-methyl-D-aspartate (NMDA) receptor ("multi-active compounds") and the use of such compounds for treating depression.

The ability of the multi-active compounds to act effectively at both the serotonin reuptake site and NMDA receptor enhances, rather than detracts, from their effectiveness. In general, potent activity at the serotonin reuptake site is favored, while an intermediate activity at the NMDA receptor is favored. Too potent an activity at the NMDA receptor is less preferred because of possible PCP-like side effects. Activity at the serotonin reuptake site and the NMDA receptor can be measured using techniques well known in the art.

Examples of assays which can be employed to measure serotonin reuptake site and NMDA receptor activities include the "serotonin reuptake inhibition assays", or "SSRI assay", the "NMDA assay", and the [$^3$H]MK-801 binding assay which are described in Example 1. Preferred compounds have an $IC_{50}$ at the serotonin reuptake site less than or equal to about 100 nM, less than' or equal to about 10 nM, or less than or equal to about 1 nM as measured by the serotonin reuptake inhibition assay.

Preferred compounds also have an $IC_{50}$ at the NMDA receptor of between about 50 nM to about 1 $\mu$M as measured by the NMDA assay. More preferably, the $IC_{50}$ at the NMDA receptor is about 100 nM to about 800 nM; and even more preferably about 500 nM.

The invention is summarized in the following items:

1. Use of a compound having the following chemical structure:

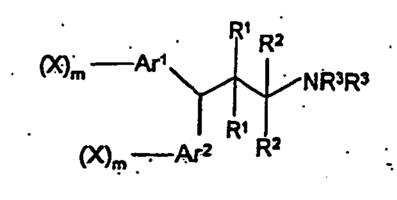

wherein each X is independently selected from the group consisting of -F, -Cl, $-OCF_3$, or $-CF_3$;
$Ar^1$ and $Ar^2$ are each independently selected from the group consisting of phenyl, naphthyl, thiofuranyl, tetrahydronaphthyl, furanyl, tetrahydrofuranyl, pyridyl, quinolinyl, isoquinolinyl, tetrahydroquinolinyl, tetrahydroisoquinolinyl, cyclohexyl, cycloheptyl, and cyclopentyl;
each $R^1$ is -H;
each $R^2$ is -H;
one $R^3$ is -H, and the other $R^3$ is either -H or $-CH_3$;
each m is 1;

provided that if one $R^3$ is -H and the other $R^3$ is $-CH_3$ then both $X_m$'s are not 4-Cl;
or a pharmaceutically acceptable salt thereof;
for the preparation of a medicament for treating a patient for depression.

2. Use of a compound having the chemical structure:

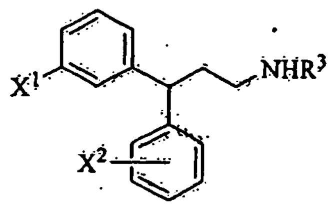

wherein $X^1$ is either -Br, -Cl, -F, -I, $-CF_3$, alkyl, -OH, $-OCF_3$, -O-alkyl, or -O-acyl;
$X^2$ is either -Br, -Cl, -F, -I, $-CF_3$, alkyl, -OH, $-OCF_3$ -O-alkyl, or -O-acyl; and
$R^3$ is either -H or $-CH_3$;
or a pharmaceutically acceptable salt thereof;
for the preparation of a medicament for treating a patient for depression.

3. Use of item 2, wherein $X^1$ is -F, -Cl, $-OCF_3$ or $-CF_3$; and $X^2$ is either 2-$OCH_3$, 2-$CH_3$, 3-F, 3-$CF_3$, or 4-$CF_3$.

By "alkyl" is meant a branched chain, straight chain; or cyclic, hydrocarbon containing between 1 and 6 carbon atoms, preferably between 1 and 4 carbon atoms. Examples of alkyl include methyl, ethyl, *n*-propyl, *iso*-propyl, *n*-butyl, sec-butyl, iso-butyl, *tert*-butyl, 2-methylpentyl, cyclopropylmethyl, and cyclobutylmethyl. Preferably, the alkyl is a branched or straight chain.
By "hydroxyalkyl" is meant an alkyl group as defined above, substituted with a hydroxyl group.
By "alkylphenyl" is meant an alkyl group as defined above, substituted with a phenyl group.
By "acyl" is meant -C(O)R, where R is H or alkyl as defined above, such as, e.g., formyl, acetyl, propionyl, or butyryl; or, R is -O-alkyl such as in alkyl carbonates or R is N-alkyl such as in alkyl carbamates.
Various examples are described herein. These examples are not intended in any way to limit the claimed invention.
Other features and advantages of the invention will be apparent from the description of the invention, the examples, and the claims.
The present invention features compounds active at both the serotonin reuptake site and the NMDA receptor and the use of such compounds for treating depression. As illustrated in the examples provided below, such compounds may also have significant activity at other sites such as the dopamine reuptake site and the norephinephrine reuptake site.

<u>Structure I Compounds</u>

Structure I compounds are as follows:

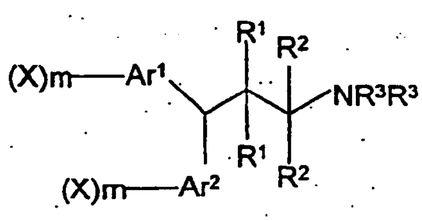

wherein each X is independently either -F, -Cl, $-OCF_3$ or $-CF_3$;
$Ar^1$ and $Ar^2$ are each independently selected from the group consisting of phenyl, naphthyl, thiofuranyl, tetrahydronaphthyl, furanyl, tetrahydrofuranyl, pyridyl, quinolinyl, isoquinolinyl, tetrahydroquinolinyl, tetrahydroisoquinolinyl, cyclohexyl, cycloheptyl, and cyclopentyl; preferably $Ar^1$ and $Ar^2$ are independently naphthyl .or phenyl; more preferably, at least one of $Ar^1$ and $Ar^2$ is phenyl; and more preferably, both $Ar^1$ and $Ar^2$ are phenyl;
each $R^1$ is -H;
each $R^2$ is -H;

one $R^3$ is -H, and the other $R^3$ is either -H or $-CH_3$; and
m is 1;
provided that if one $R_3$ is -H and the other $R^3$ is $-CH_3$ then both $X_m$'s are not 4-Cl;
or a pharmaceutically acceptable salt thereof.

Substitutions in both the Structure I upper and lower phenyl rings are useful for providing serotonin reuptake site and NMDA receptor activities. The effect of different substitution patterns is illustrated in the data provided in Example 1.

An embodiment is provided by Structure IV compounds as follows :

Structure IV

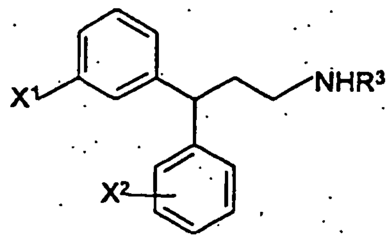

wherein $X^1$ is either -Br, -Cl, -F, -I, $-CF_3$, alkyl, -OH, $-OCF_3$, -O-alkyl, or -O-acyl; preferably, $X^1$ is either -F, -Cl, $-OCF_3$ or $-CF_3$; and more preferably $X^1$ is -F;
$X^2$ is either -Br, -Cl, -F, -I, $-CF_3$, alkyl, -OH, $-OCF_3$, -O-alkyl, or -O-acyl; preferably, $X^2$ is independently either -F, -Cl, $-OCH_3$, $-CH_3$, $-OCF_3$ or $-CF_3$; more preferably, $X^2$ is either 2-$OCH_3$, 2-$CH_3$, 3-F, 3-$CF_3$, or 4-$CF_3$; and
$R^3$ is either -H or $CH_3$;
or a pharmaceutically acceptable salt thereof.

Certain compounds are presented below in Table I. Examples of certain antidepressant compounds are shown in Table II. Compounds 20, 60, and 142 are useful in the present invention.

Table I

| Comp. | Structure | fura2data $IC_{50}$ (μM) | [$^3$H]MK-801 data $IC_{50}$ (μM) | 5-HT Uptake | NE Uptake | DA Uptake |
|---|---|---|---|---|---|---|
| 19 | 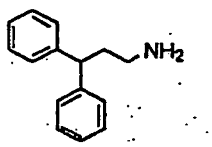 | 0.435 | 2.1 | 18.2<br>89.2 | 2.8<br>52.6 | 6.7.<br>75.0 |
| 20 | 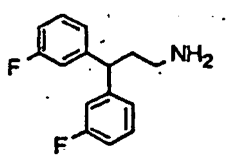 | 0.070 | 0.252 | $K_i$ = 0.256 | $K_i$ = 0.312 | $K_i$ = 3.0 |
| 33 | 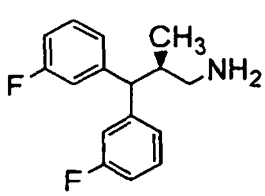 | 0.060 | 0.181 | $K_i$ = 1.2 | $K_i$ = 3.8 | $K_i$ = 0.826 |

(continued)

| Comp. | Structure | fura2data $IC_{50}$ (μM) | [$^3$H]MK-801 data $IC_{50}$ (μM) | 5-HT Uptake | NE Uptake | DA Uptake |
|---|---|---|---|---|---|---|
| 34 | 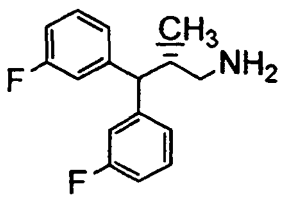 | 0.426 | 2.7 | $K_i$ = 2.1 | $K_i$ = 3.0 | $K_i$ = 0.602 |
| 50 | 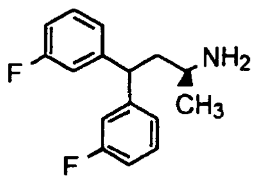 | 0.089 | 0.762 | $K_i$ = 0.0 | $K_i$ = 0.107 | $K_i$ = 1.8 |
| 46 | 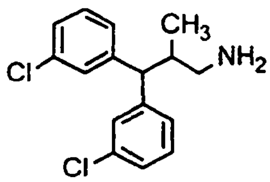 | 0.013 | 5.2 | 10.4<br>75.4 | 7.4<br>64.0 | 40.2<br>97.1 |
| 236 | 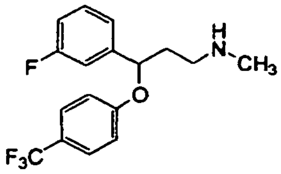 | | | | | |
| 237 | 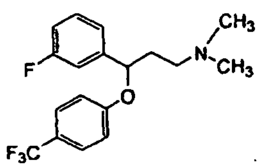 | | | | | |
| 63 | 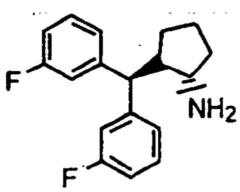 | 0.093 | 0.245 | 13.4<br>84.6 | 6.8<br>76.2 | 64.3<br>97.8 |
| 58 | 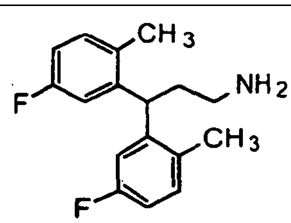 | 0.028 | 0.203 | 27.2<br>95.2 | 1.3<br>66.6 | 0.0<br>45.2 |
| 59 | 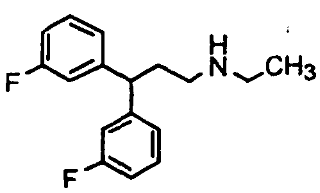 | 0.272 | 0.453 | 14.3<br>85.7 | 7.4<br>50.6 | 1.6<br>77.6 |

(continued)

| Comp. | Structure | fura2data $IC_{50}$ ($\mu$M) | [$^3$H]MK-801 data $IC_{50}$ ($\mu$M) | 5-HT Uptake | NE Uptake | DA Uptake |
|---|---|---|---|---|---|---|
| 64 | 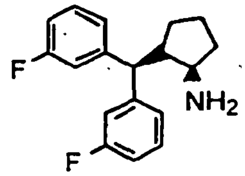 | 0.309 | 0.851 | $K_i$ = 0.25 | $K_i$ = 0.508 | $K_i$ = 0.119 |
| 60 | 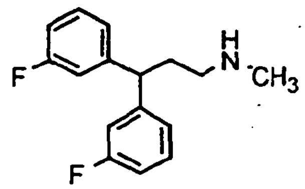 | 0.416 | 0.641 | $K_i$ = 0.06 | $K_i$ > 10.0 | $K_i$ = 0.914 |
| 65 | 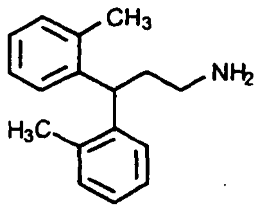 | 0.167 | 2.0 | $K_i$ = 0.12 | $K_i$ = 0.306 | $K_i$ = 22.0 |
| 66 | 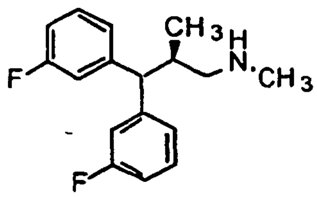 | 0.236 | 1.2 | 6.1<br>67.5 | 4.2<br>36.5 | 4.4<br>92.8 |
| 67 | 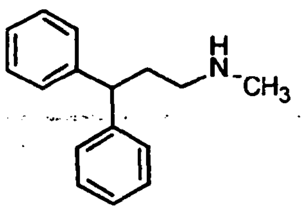 | 10.95 | 2.9 | | | |
| 69 | 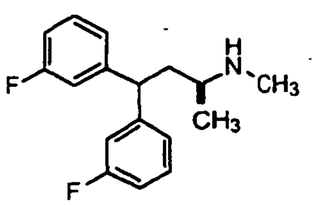 | 0.224 | 0.366 | | | |
| 108 | 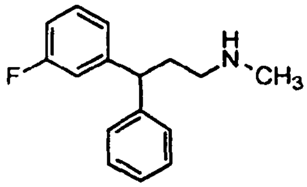 | 1.06 | 0.942 | | | |

(continued)

| Comp. | Structure | fura2data $IC_{50}$ ($\mu$M) | [$^3$H]MK-801 data $IC_{50}$ ($\mu$M) | 5-HT Uptake | NE Uptake | DA Uptake |
|---|---|---|---|---|---|---|
| 111 | 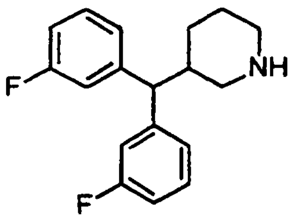 | 0.645 | 0.167 | $K_i$ = 0.600 | $K_i$ = 0.124 | $K_i$ = 0.030 |
| 216 | 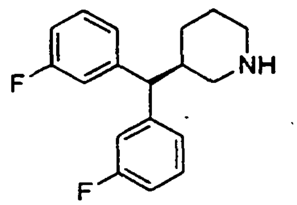 | | | | | |
| 217 | 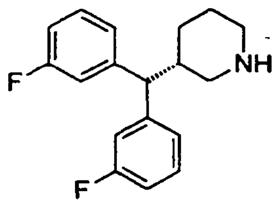 | | | | | |
| 118 | 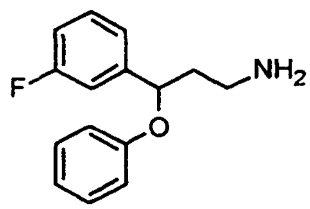 | 0.409 | 0.240 | $K_i$ = 0.131 | $K_i$ = 0.262 | $K_i$ = 4.1 |
| N-methyl-118 | 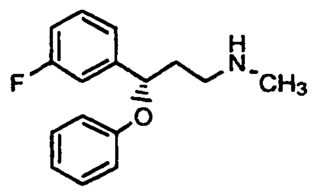 | | | | | |
| N-methyl-118 | 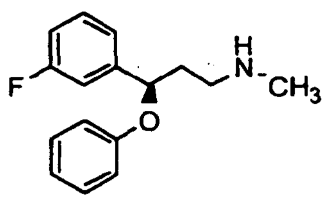 | | | | | |
| 119 | 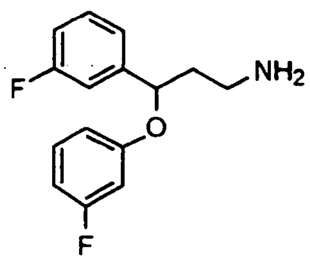 | 0.115 | 0.087 | 0.6<br>3.1 | 8.9<br>19.4 | 1.9<br>58.3 |

(continued)

| Comp. | Structure | fura2data $IC_{50}$ ($\mu$M) | [$^3$H]MK-801 data $IC_{50}$ ($\mu$M) | 5-HT Uptake | NE Uptake | DA Uptake |
|---|---|---|---|---|---|---|
| 120 | 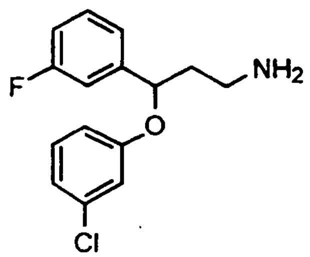 | 0.101 | 0.074 | | | |
| 121 | 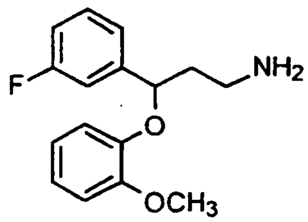 | 0.656 | 0.670 | 8.5<br>84.3 | 33.6<br>94.9 | 1.8<br>91.3 |
| 122 | 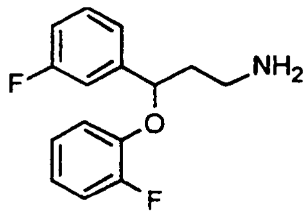 | 0.209 | 0.342 | $K_i$ = 0.062 | $K_1$ = 0.103 | $K_i$ = 2.8 |
| 137 | 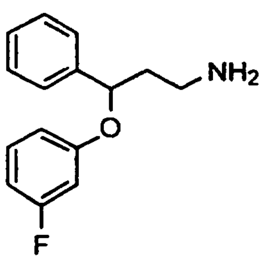 | 0.232 | 0.074 | | | |
| 221 | 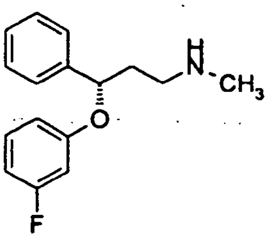 | | | | | |
| 222 | 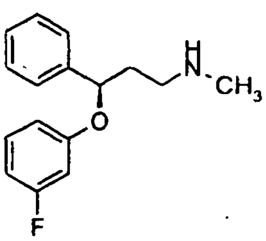 | | | | | |

(continued)

| Comp. | Structure | fura2data $IC_{50}$ (μM) | [$^3$H]MK-801 data $IC_{50}$ (μM) | 5-HT Uptake | NE Uptake | DA Uptake |
|---|---|---|---|---|---|---|
| 142 | 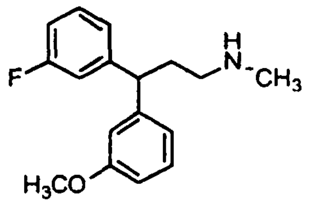 | 1.013 | 1.54 | | | |
| 144 | 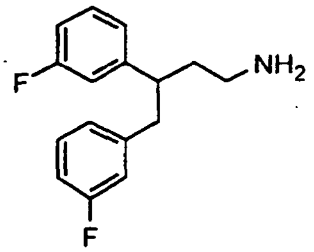 | | | | | |
| 145 | 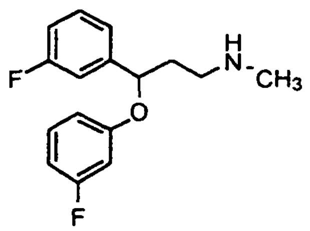 | 0.098 | 0.626 | 3.5<br>81.1 | 16.6<br>99.4 | 0.0<br>87.4 |
| 148 | 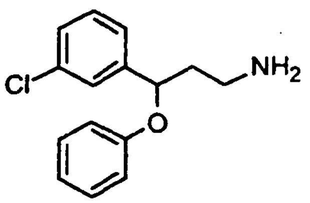 | 0.549 | 0.373 | | | |
| 149 | 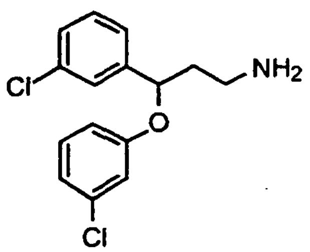 | 0.085 | 0.150 | | | |
| 150 | 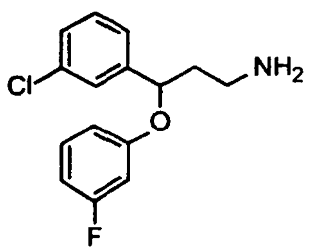 | 0.195 | 0.351 | | | |

(continued)

| Comp. | Structure | fura2data $IC_{50}$ ($\mu M$) | [$^3$H]MK-801 data $IC_{50}$ ($\mu M$) | 5-HT Uptake | NE Uptake | DA Uptake |
|---|---|---|---|---|---|---|
| 156 | 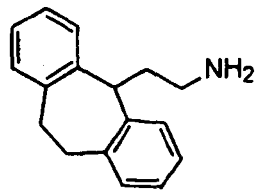 | 0.069 | 0.090 | $K_1$ = 1.2 | $K_i$ = 0.570 | $K_i$ >10.0 |
| 177 | 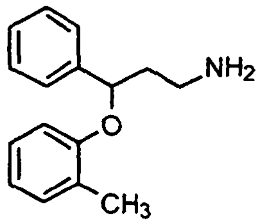 | | 0.754 | $K_1$ = 0.056 | $K_i$ = 0.109 | $K_i$ >10.0 |
| 178 | 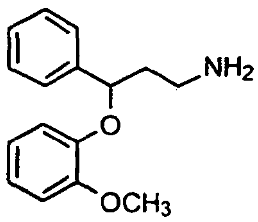 | | 1.25 | | | |
| 179 | 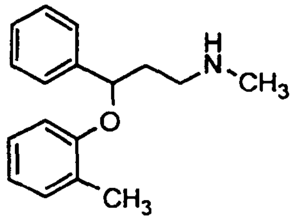 | | 1.67 | | | |
| 181 | 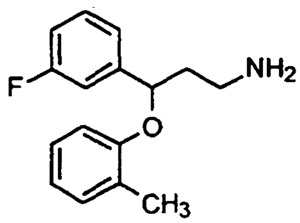 | 0.081 | 0.632 | 37.4<br>74.2 | 10.7<br>32.0 | 0.0<br>108.8 |
| 182 | 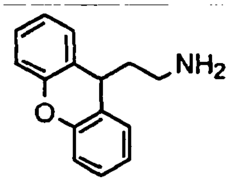 | 2.6 | 7.05 | | | |
| 183 | 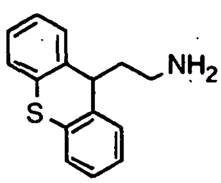 | 0.676 | 5.01 | | | |

(continued)

| Comp. | Structure | fura2data $IC_{50}$ (μM) | [3H]MK-801 data $IC_{50}$ (μM) | 5-HT Uptake | NE Uptake | DA Uptake |
|---|---|---|---|---|---|---|
| 184 | 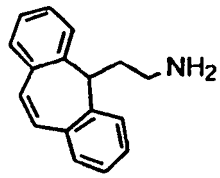 | 1.5 | 1.51 | | | |
| 185 | 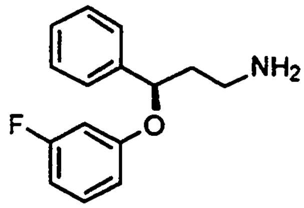 | 0.646 | 0.639 | $K_i$ = 2.4 | $K_i$ = 0.858 | $K_i$ = 1.1 |
| 186 | 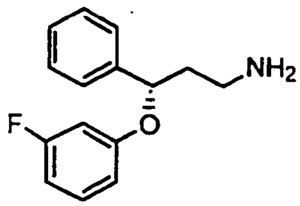 | 0.155 | 0.123 | $K_i$ = 0.022 | $K_i$ = 0.136 | $K_i$ = 3.1 |
| 187 | 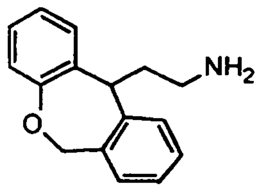 | 1.78 | 2.01 | | | |
| 191 | 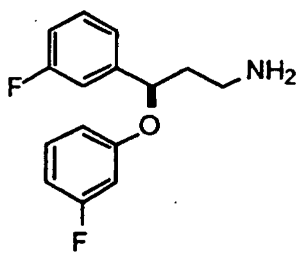 | | 1.3 | $K_i$ = 0.134 | $K_i$ = 0.638 | $K_i$ = 1.55 |
| 192 | 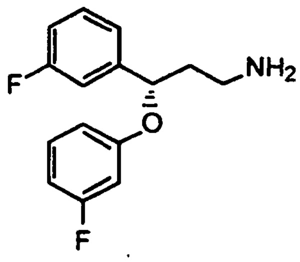 | | 0.111 | $K_i$ = 0.014 | $K_i$ = 0.325 | $K_i$ = 1.3 |

(continued)

| Comp. | Structure | fura2data $IC_{50}$ (μM) | [3H]MK-801 data $IC_{50}$ (μM) | 5-HT Uptake | NE Uptake | DA Uptake |
|---|---|---|---|---|---|---|
| 193 | 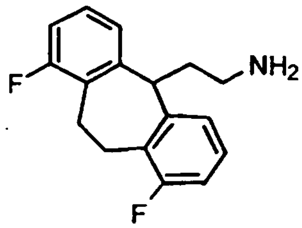 | | >1.0 | | | |
| 194 | 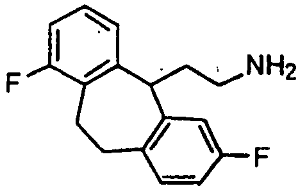 | | 0.371 | | | |
| 195 | 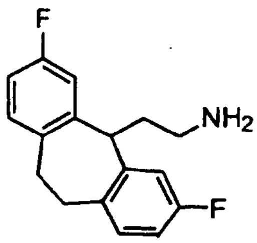 | | 0.029 | | | |
| 196 | 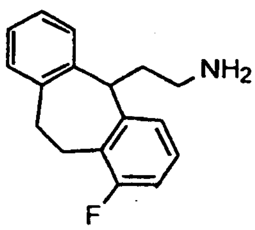 | | 2.24 | | | |
| 197 | 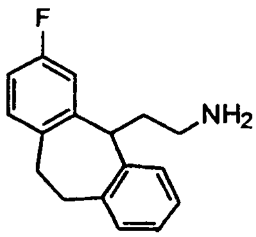 | | 0.053 | 9.8<br>58.1 | 0.0<br>27.5 | 0.0<br>83.5 |
| 198 | 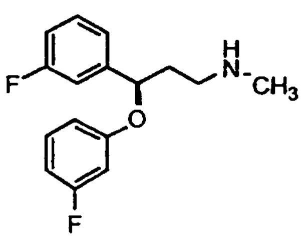 | | | | | |

(continued)

| Comp. | Structure | fura2data $IC_{50}$ (μM) | [$^3$H]MK-801 data $IC_{50}$ (μM) | 5-HT Uptake | NE Uptake | DA Uptake |
|---|---|---|---|---|---|---|
| 199 | 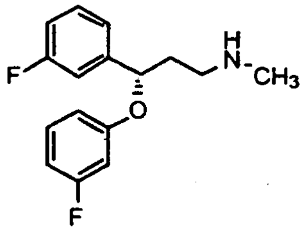 | | | | | |
| 218 | 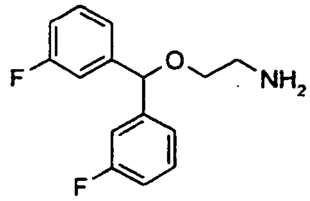 | | | | | |
| 219 | 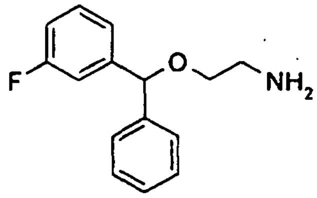 | | | | | |
| 220 | 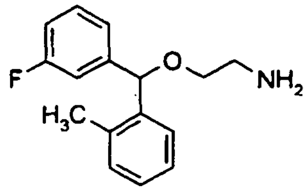 | | | | | |
| 224 | 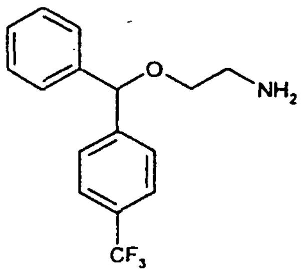 | | | | | |
| 225 | 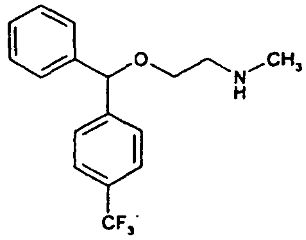 | | | | | |

(continued)

| Comp. | Structure | fura2data $IC_{50}$ (μM) | [$^3$H]MK-801 data $IC_{50}$ (μM) | 5-HT Uptake | NE Uptake | DA Uptake |
|---|---|---|---|---|---|---|
| 226 | 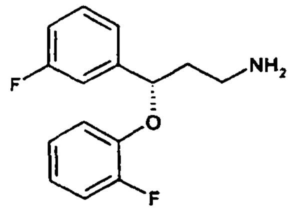 | | | | | |
| | 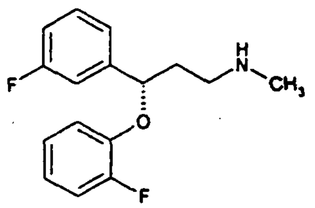 | | | | | |
| 228 | 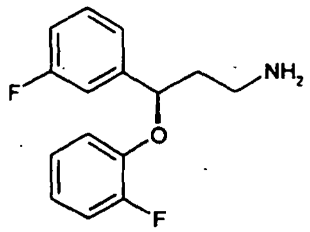 | | | | | |
| 230 | 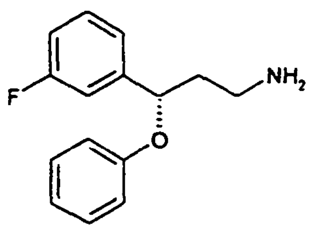 | | | | | |
| 231 | 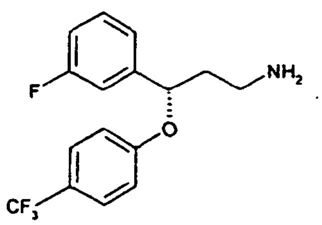 | | | | | |
| 232 | 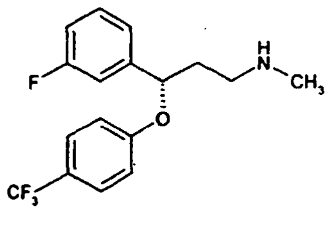 | | | | | |

(continued)

| Comp. | Structure | fura2data $IC_{50}$ (μM) | [$^3$H]MK-801 data $IC_{50}$ (μM) | 5-HT Uptake | NE Uptake | DA Uptake |
|---|---|---|---|---|---|---|
| 233 | 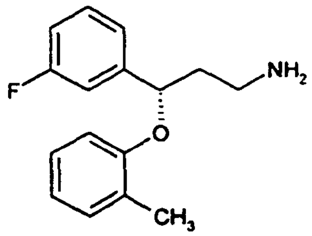 | | | | | |
| 234 | 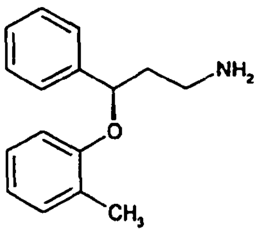 | | | | | |
| 235 | 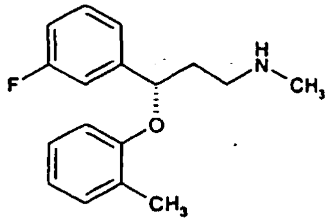 | | | | | |
| 229 | 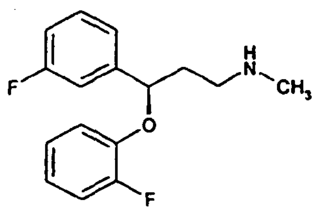 | | | | | |

top numbers for 5-HT (serotonin assay), NE (norepinephrine assay), and DA (dopamine assay) refer to mean % inhibition (±SEM) @ 100 nM, (except where $K_i$ values (μM) are indicated)
bottom numbers for 5HT, NE, and DA refer to mean % inhibition (±SEM) @ 10.0 μM, (except where $K_i$ [μM] values are indicated).

Table II

| Comp. | Structure | fura2 data IC50 (μM) | [$^3$H]MK-801 data | 5-HT Uptake | NE Uptake | DA Uptake |
|---|---|---|---|---|---|---|
| Fluoxetine | 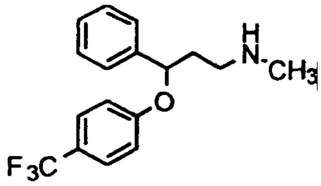 | 3.4 | No activity at 10 μM | > 60 %<br>99 % | 0<br>68 | 0<br>78 |

(continued)

| Comp. | Structure | fura2 data IC50 (μM) | [3H]MK-801 data | 5-HT Uptake | NE Uptake | DA Uptake |
|---|---|---|---|---|---|---|
| Paroxetine | 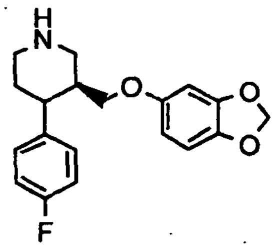 | | | > 80 %<br>98 % | <20<br>50 | <20<br>97 |
| Desipramine | 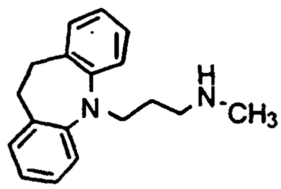 | 2.3 | | <20<br>90 | <20<br>72 | <20<br>64 |
| Bupropion | 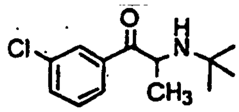 | | | n.t.<br><20 | n.t.<br><20 | <20<br>99 |
| Phenylphenoxy -propylamine | 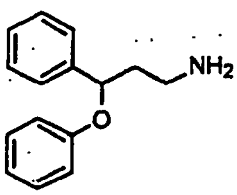 | 0.829 | 0.372 | | | |
| Nisoxetine | 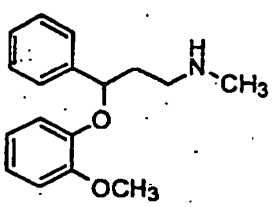 | 1.23 | | | | |
| Flunamine | 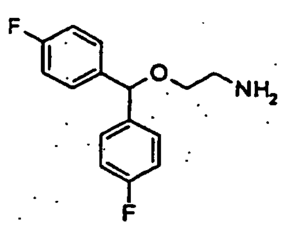 | | | | | |

top numbers for 5-HT (serotonin assay), NE (norepinephrine assay), and DA (dopamine assay) refer to mean % inhibition (±SEM) @ 100 nM.
bottom numbers for SHT, NE, and DA refer to mean % inhibition (±SEM) @ 10.0 μM.
n.t. indicates not tested.

Administration

The methods and compounds will typically be used in therapy for human patients. However, they may also be

used to treat depression in other vertebrates such as other primates, sports animals, and pets such as horses, dogs and cats.

Suitable dosage forms, in part, depend upon the use or the route of administration, for example, oral, transdermal, transmucosal, or by injection (parenteral). Such dosage forms should allow the compound to reach target cells. Other factors are well known in the art, and include considerations such as toxicity and dosage forms that retard the compound or composition from exerting its effects. Techniques and formulations generally may be found in Remington's Pharmaceutical Sciences, $18^{th}$ ed., Mack Publishing Co., Easton, PA, 1990.

Compounds can be formulated as pharmaceutically acceptable salts. Pharmaceutically acceptable salts are non-toxic salts in the amounts and concentrations at which they are administered. The preparation of such salts can facilitate the pharmacological use by altering the physical characteristics of a compound without preventing it from exerting its physiological effect. Useful alterations in physical properties include lowering the melting point to facilitate transmucosal administration and increasing the solubility to facilitate administering higher concentrations of the drug.

Pharmaceutically acceptable salts include acid addition salts such as those containing sulfate, chloride, hydrochloride, fumarate, maleate, phosphate, sulfamate, acetate, citrate, lactate, tartrate, methanesulfonate, ethanesulfonate, benzenesulfonate, *p*-toluenesulfonate, cyclohexylsulfamate and quinate. Pharmaceutically acceptable salts can be obtained from acids such as hydrochloric acid, maleic acid, sulfuric acid, phosphoric acid, sulfamic acid, acetic acid, citric acid, lactic acid, tartaric acid, malonic acid, methanesulfonic acid, ethanesulfonic acid, benzenesulfonic acid, *p*-toluenesulfonic acid, cyclohexylsulfamic acid, fumaric acid, and quinic acid.

Pharmaceutically acceptable salts also include basic addition salts such as those containing benzathine, chloroprocaine, choline, diethanolamine, ethylenediamine, meglumine, procaine, aluminum, calcium, lithium, magnesium, potassium, sodium, ammonium, alkylamine, and zinc, when acidic functional groups, such as carboxylic acid or phenol are present. For example, see Remington's Pharmaceutical Sciences, $19^{th}$ ed., Mack Publishing Co., Easton, PA, Vol. 2, p. 1457, 1995. Such salts can be prepared using the appropriate corresponding bases.

Pharmaceutically acceptable salts can be prepared by standard techniques. For example, the free-base form of a compound is dissolved in a suitable solvent, such as an aqueous or aqueous-alcohol in solution containing the appropriate acid and then isolated by evaporating the solution. In another example, a salt is prepared by reacting the free base and acid in an organic solvent.

The pharmaceutically acceptable salt of the different compounds may be present as a complex. Examples of complexes include 8-chlorotheophylline complex (analogous to, *e.g.*, dimenhydrinate: diphenhydramine 8-chlorotheophylline (1:1) complex; Dramamine) and various cyclodextrin inclusion complexes.

Carriers or excipients can be used to produce pharmaceutical compositions. The carriers or excipients can be chosen to facilitate administration of the compound. Examples of carriers include calcium carbonate, calcium phosphate, various sugars such as lactose, glucose, or sucrose, or types of starch, cellulose derivatives, gelatin, vegetable oils, polyethylene glycols and physiologically compatible solvents. Examples of physiologically compatible solvents include sterile solutions of water for injection (WFI), saline solution, and dextrose.

The compounds can be administered by different routes including intravenous, intraperitoneal, subcutaneous, intramuscular, oral, transmucosal, rectal, or transdermal. Oral administration is preferred. For oral administration, for example, the compounds can be formulated into conventional oral dosage forms such as capsules, tablets, and liquid preparations such as syrups, elixirs, and concentrated drops.

Pharmaceutical preparations for oral use can be obtained, for example, by combining the active compounds with solid excipients, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations, for example, maize starch, wheat starch, rice starch, potato starch, gelatin, gum tragacanth, methyl cellulose, hydroxypropylmethyl-cellulose, sodium carboxymethylcellulose (CMC), and/or polyvinylpyrrolidone (PVP: povidone). If desired, disintegrating agents may be added, such as the cross-linked polyvinylpyrrolidone, agar, or alginic acid, or a salt thereof such as sodium alginate.

Dragee cores are provided with suitable coatings. For this purpose, concentrated sugar solutions may be used, which may optionally contain, for example, gum arabic, talc, polyvinylpyrrolidone, carbopol gel, polyethylene glycol (PEG), and/or titanium dioxide, lacquer solutions, and suitable organic solvents or solvent mixtures. Dye-stuffs or pigments may be added to the tablets or dragee coatings for identification or to characterize different combinations of active compound doses.

Pharmaceutical preparations that can be used orally include push-fit capsules made of gelatin ("gelcaps"), as well as soft, sealed capsules made of gelatin, and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols (PEGs). In addition, stabilizers may be added.

Alternatively, injection (parenteral administration) may be used, *e.g.*, intramuscular, intravenous, intraperitoneal, and/orsubcutaneous. For injection, the compounds of the invention are formulated in sterile liquid solutions, preferably in physiologically compatible buffers or solutions, such as saline solution, Hank's solution, or Ringer's solution. In addition, the compounds may be formulated in solid form and redissolved or suspended immediately prior to use. Lyophilized forms can also be produced.

Administration can also be by transmucosal or transdermal means. For transmucosal or transdermal administration, penetrants appropriate to the barrier to be permeated are used in the formulation. Such penetrants are generally known in the art, and include, for example, for transmucosal administration, bile salts and fusidic acid derivatives. In addition, detergents may be used to facilitate permeation. Transmucosal administration, for example, may be through nasal sprays or suppositories (rectal or vaginal).

The amounts of various compound to be administered can be determined by standard procedures taking into account factors such as the compound $IC_{50}$, the biological half-life of the compound, the age, size, and weight of the patient, and the disorder associated with the patient. The importance of these and other factors are well known to those of ordinary skill in the art. Generally, a dose will be between about 0.01 and 50 mg/kg, preferably 0.1 and 20 mg/kg of the patient being treated. Multiple doses may be used.

Additional examples are provided below illustrating different aspects and embodiments of the present invention. These examples are not intended in any way to limit the disclosed invention, which is defined by the claims.

Example 1: Pharmacological Effects of Different Compounds

This example illustrates the activity of different compounds at different monoamine reuptake sites and the NMDA receptor. Table I illustrates the activities at different monoamine reuptake sites and the NMDA receptor, and compounds expected to have significant activity at the serotonin reuptake site and the NMDA receptor. Table II provides the activities of different anti-depressants at different monoamine reuptake sites and the NMDA receptor.

*MONOAMINE TRANSPORTER BINDING ASSAYS*

Monoamine transporter binding assays (reuptake inhibition assays) were performed by NovaScreen using standard radioligand binding assays. The transporter binding assays are described briefly below:

Serotonin Reuptake Inhibition Assay

Rat forebrain membranes were incubated with 0.7 nM [$^3$H] citalopram, N-methyl, (70-87 Ci/mmol) in 50 mM Tris-HCl (pH 7.4), 120 mM NaCl, and 5 mM KCl at 25 ˚C for 60 minutes. Nonspecific binding was determined using 10 μM clomipramine and imipramine was used as the positive control reference compound. Reactions were terminated by rapid vacuum filtration onto glass fiber filters. Bound radioactivity was determined using liquid scintillation spectrometry and experimental values were compared to control values to determine binding to the serotonin transporter site (based on D'Amato, *R.J. et al., J. Pharmacol. Exp. Ther., 242*, 364-371, 1987, and Brown, *N.L. et al., Eur. J. Pharmacol., 123*, 161-165, 1986).

Norepinephrine Reuptake Inhibition Assay

Rat forebrain membranes were incubated with 1.0 nM [$^3$H] nisoxetine (60-85 Ci/mmol) in 50 mM Tris-HCl (pH 7.4), 300 mM NaCl, and 5 mM KCl at 0-4 ˚C for 4 hours. Nonspecific binding was determined using 1.0 μM desipramine and desipramine was used as the positive control reference compound. Reactions were terminated by rapid vacuum filtration onto glass fiber filters. Bound radioactivity was determined using liquid scintillation spectrometry and experimental values were compared to control values to determine binding to the norepinephrine transporter site (Raisman, R. *et al., Eur. J. Pharmacol., 78*, 345-351, 1982, and Langer, S.Z. *et al., Eur. J. Pharmacol.*, *72*, 423-424, 1981).

Dopamine Reuptake Inhibition Assay :

Guinea pig striatal membranes were incubated with 2.0 nM [$^3$H] WIN 35428 (60-87 Ci/mmol)in 50 mM Tris-HC (pH 7.4), 120 mM NaCl at 0-4 ˚C for 2 hours. Nonspecific binding was determined using 1.0 μM GBR-12909 and GBR-12909 was used as the positive control reference compound. Reactions were terminated by rapid vacuum filtration onto glass fiber filters. Bound radioactivity was determined using liquid scintillation spectrometry and experimental values were compared to control values to determine binding to the dopamine transporter site (based on Madras, *et al., Molec. Pharmacol., 36,* 518-524, 1989, and Javitch, J.J., *et al., Molec. Pharmacol., 26,* 35-44 1984).

*NMDA RECEPTOR ASSAY*

Reagents

All culture media, antibiotics, and enzymes, with the exception of fetal calf serum (Hyclone Laboratories, Logan, UT), were purchased from Sigma Chemical Co., St. Louis, MO. Fura-2/AM was obtained from Molecular Probes, Eugene, OR, and was prepared freshly in dimethylsulfoxide (DMSO) before use. Ionomycin (Calbiochem) was stored as a stock solution in DMSO. Nifedipine (Sigma) and nimodipine (RBI) were dissolved in absolute ethanol. The final concentration of ethanol in the cuvette never exceeded 0.05% and was without effect on basal cytosolic calcium ($[Ca^{2+}]_i$). All other agents were dissolved in phosphate-buffered saline (PBS), and adjusted to pH 7.4

Preparation of Rat Cerebellar Granule Cell (RCGC) Cultures

A simple and rapid method for measuring $[Ca^{2+}]_i$ in large homogeneous populations of normal central nervous system neurons has been described in detail (Parks *et al.,* Modulation of N-methyl-D-aspartate receptor-mediated increases in cytosolic calcium in cultured rat cerebellar granule cells. *Brain Res. 552*: 13-22, 1991). Briefly, primary cultures of cerebellar granule neurons were obtained from 8-day-old rats and plated onto squares of Aclar plastic coated with poly-L-lysine. The plastic squares were placed in 12-well culture plates, and ~7.5 x $10^5$ cells were added to each well. Cultures were maintained in Eagles's medium containing 25 mM KCl, 10% fetal calf serum, 2 mM glutamine, 100 μg/ml gentamicin, 50 U/ml penicillin, and 50 ug/ml streptomycin at 37 ˚C in a humid atmosphere of 5% $CO_2$ in air for 24 hr before the addition of cytosine arabinoside (10 μM, final). No changes of culture medium were made until the cells were used for fluorescence recording 6-8 days after plating.

Measurement of Cytosolic Calcium

For measurement of $[Ca^{2+}]_i$, Aclar squares plated with cells were incubated in a HEPES buffer containing 125 mM NaCl, 5 mM KCl, 1.5 mM $CaCl_2$, 5.6 mM glucose, 25 mM NaHEPES (pH 7.4), 0.1% bovine serum albumin and 2 μM fura-2/acetoxymethylester (fura-2/AM) for 30-40 min at 37 ˚C ("$Ca^{2+}$-free" buffer contained no added $CaCl_2$ and 30 μM EGTA). The cells were then rinsed with the same buffer, lacking fura-2/AM, and maintained at room temperature until used for measurement of fluorescence, the Aclar squares were transferred into quartz cuvettes containing 2 ml of the HEPES buffer. The cuvette was then placed into a thermostatted holder equipped with a magnetic stirrer in a custom-built spectrofluorimeter (Biomedical Instrumentation Group, University of Pennsylvania, Philadelphia, PA). The size of the plastic Aclar square was such as to fit snugly in the cuvette when placed at a diagonal and therefore suspend itself over the spin bar. Fluorescence was measured using excitation and emission wavelengths of 340 and 510 nm, respectively. The amplitude of the evoked increase in fluorescence and subsequent inhibition were recorded and the concentration of $[Ca^{2+}]_i$ was calculated using the formula:

$$[Ca^{2+}] = K_d\ (F-F_{min})/(F_{max}-F) \quad \text{(Eq. 1)}$$

where:

F = fluorescence amplitude evoked at a particular time point or concentration of compound;
$F_{min}$ = minimum fluorescence determined after the addition of a 2.5 M TRIS/0.3 M EGTA solution;
$F_{max}$ = maximum fluorescence determined following the addition of 7 mM ionomycin in DMSO; and
$K_d$ = dissociation constant for fluorometric indicator (for fura-2, the $K_d$ = 224 nM).
Fluorescent signals were calibrated by adding ionomycin (35-42 μM, final) to obtain $F_{max}$ and EGTA (30-12 mM, final, pH 8.2) to obtain $F_{min}$.

*[$^3$H] MK- 801 BINDING ASSAY*

Synaptic plasma membranes (SPMs) from rat cortex were prepared as follows. Isolated cerebral cortices from male and female rats were purchased from Pel-Freez (Rogers, AZ) in bulk, and stored at -80 ˚C. The cortices from 25 rats were thawed and pooled. Tissues were homogenized at 4 ˚C with a polytron (ESGE Biohomogenizer, #133/1281-0) for 10 pulses at the highest setting in 300 ml of 0.32 M sucrose containing 5 mM K-EDTA (pH 7.0). The resulting

homogenates were centrifuged for 10 min at 1,000 x *g* in a T865 rotor, UltraPro80 Sorvall Centrifuge. The supernatant was removed and subsequently centrifuged at 30,000 x *g* for 30 min. The resulting pellets were resuspended in 250 ml of 5 mM K-EDTA (pH 7.0), stirred on ice for 15 min, and then centrifuged at 30,000 x *g* for 30 min. Membranes were washed by re-suspension in 500 ml of 5 mM K-EDTA (pH 7.0), incubated at 32 °C for 30 min with stirring, and centrifuged at 100,000 x *g* for 30 min. The final pellet was resuspended in 60 ml of 5 mM K-EDTA (pH 7.0) and stored in aliquots at -80 °C. The extensive washing procedure utilized in this assay was designed to minimize the concentrations of glutamate and glycine (co-agonists at the NMDA receptor-ionophore complex) present in the membrane preparation.

On the day of assay, aliquots of SPM were thawed and resuspended in 75 vols of 5 mM K-EDTA, pH 7.0. SPM were centrifuged at 100,000 x *g* for 30 min at 4 °C.

Displacement studies were carried out as follows (Williams *et al.,* Effects of polyamines on the binding of [3H] MK-801 to the NMDA receptor: pharmacological evidence for the existence of a polyamine recognition site. *Mol. Pharmacol.* 36: 575-581, 1989). The SPM pellet was resuspended in assay buffer (30 mM EPPS, 1 mM K-EDTA, pH 7.0) by polytron. MK-801 binding assays were carried out in a incubation volume of 0.5 ml containing 80-100 μg of membrane protein per tube. Duplicate samples were incubated for 2-3 hr at 25°C with < 5 nM [$^3$H] MK-801, 100 μM glycine, 100 μM L-glutamic acid and varying concentrations of displacer. Non-specific binding was determined by the inclusion of 10 μM ketamine or MK-801. Binding was started by the addition of the tissue homogenate. The assay was terminated by the addition of 4 ml ice-cold buffer, followed by filtration over glass-fiber filters (Schleicher & Schuell No. 30) on a Millipore 12-well filtration manifold. Filters were washed with another 3 x 4 ml ice-cold buffer (pH 7.0). Radioactivity on the filters was measured using Fisherbrand Scinti-Safe scintillation cocktail. Samples were shaken for 45-60 min on a shaker platform to solubilize the radioactivity. Counting for $^3$H was performed in a Beckman 6000IC LS Scintillation Counter for 4 min per sample. Protein was determined as described by Lowry *et al.* (Protein measurement with the folin phenol reagent. *J. Biol. Chem. 193*: 265-275, 1951.).

Example 2: Antidepressant Activity

Compound 19 Antidepressant Activity

The antidepressant activity of Compound 19 was demonstrated in mice using the tail-suspension test (Steru *et al.,* The Automated Tail Suspension Test: A Computerized Device which Differentiates Psychotropic Drugs, *Prog. Neuro-Psychopharmacol. Biol. Psychiatry* 11: 659-671, 1987). In this test, the animal is suspended by the tail for 6 minutes. The behavior of the animal is recorded automatically using a special computerized apparatus which measures two parameters, the duration of immobility and the power of the movements. Ten animals are studied per dose group. Desipramine is used as a reference compound. This test, which is a variant of the behavioral despair test, is based on the assumption that animals faced with an insoluble aversive situation will alternate between phases of activity (searching to escape) and immobility (waiting, "despair").

Compound 19 was dispersed in a 5% acacia gum suspension, and administered intraperitoneally (i.p.) to male NMRI-CERJ mice, 30 min prior to testing, at the single dose of 16 mg/kg. This dose of Compound 19 elicited an 84% decrease in the duration of immobility ($p < 0.001$) and a 275% increase in the power of movements ($p < 0.001$). By comparison, the positive control desipramine (32 mg/kg) produced a 63% decrease in the duration of immobility ($p < 0.001$) and a 71% increase in the power of movements ($p > 0.05$).

Compound 60 Antidepressant Activity

The antidepressant activity of Compound 60 was demonstrated in the forced-swim test (FST) in the mouse and the rat (Porsolt *et al.,* Behavioral Despair in Mice: A Primary Screening Test for Antidepressants, *Arch. Int. Pharmacodyn.* 229: 327-336, 1977; Porsolt *et al.,* Behavioral Despair in Rats: A New Model Sensitive to Antidepressant Treatments, *Eur. J. Pharmacol.* 47: 379-391, 1978) and in the mouse tail-suspension test (TST).

For the mouse and rat forced-swim tests, male pathogen-free NIH-Swiss mice (Harlan Sprague-Dawley) weighing between 22-25 g, and male pathogen-free Sprague-Dawley rats (Harlan Sprague-Dawley) weighing between 320-350 g were used. Animals were removed from the housing room to the testing area in their own cages and allowed to adapt to the new environment for at least 1 hour before testing. Mice were administered either drug (imipramine, 10 mg/kg i.p.; or Compound 60, various p.o or i.p. doses) or saline. Thirty minutes to 24 hours later, mice were placed individually in cylinders (diameter: 10 cm; height: 25 cm) filled with 6 cm of water (22-25°C) for 6 minutes. The duration of immobility during the last 4 minutes of the 6 minute test was scored. A mouse was recorded as immobile when floating motionless or making only those movements necessary to keep its head above water. Results were expressed as the duration of immobility (seconds).

The rat forced-swim test was conducted over two days. On treatment Day 1, rats were placed individually in cylinders (diameter: 18 cm; height: 40 cm) filled with water (22-25°C) to a depth of 15 cm for 15 minutes. The duration

of immobility during the first 5 minutes was scored. After testing, the rats were dried with paper towels and placed in holding cages. Five minutes after removal from the testing cylinders, animals received either drug (imipramine, 10 mg/kg i.p.; or Compound 60, various p.o. doses) or saline (p.o.). Ten minutes later, the rats were returned to their home cages. On treatment Day 2, the animals received a second dose of drug or saline, and 1 hour later were placed in the testing cylinders as described above. The duration of immobility during the first 5 minute period was recorded. A rat was recorded as immobile when floating motionless or making only those movements necessary to keep its head above water. Results were expressed as a difference score (duration of immobility on Day 1 minus duration of immobility on Day 2).

For the mouse tail-suspension test with Compound 60, male pathogen-free C57BL/6 mice (Harlan Sprague-Dawley) weighing between 22-25 g were used. Animals were removed from the housing room to the testing area in their own cages and allowed to adapt to the new environment for at least 1 hour before testing. Mice were administered either drug (imipramine, 15 mg/kg i.p.; or Compound 60, various p.o. doses) or saline. Thirty minutes to 24 hours later, the mice were suspended on the edge of a shelf 80 cm above the floor in the testing room by adhesive tape placed approximately 1 cm from the tip of the tail. The duration of immobility was recorded during a test period of 5 minutes. Mice were considered immobile only when they hung passively and completely motionless. Results were expressed as the duration of immobility (sec).

In the mouse forced-swim test, a single dose of Compound 60, 5 mg/kg p.o., produced a time-dependent reduction in the duration of immobility (Table III), with the peak effect occurring 60 minutes post-dosing. When administered orally 60 minutes before testing, Compound 60 produced a dose-dependent reduction in the duration of immobility (Table IV). The magnitude of the antidepressant-like activity produced by Compound 60 was similar to that elicited by imipramine, 10 mg/kg i.p. (Table IV). Similar antidepressant-like activity was observed when Compound 60 was administered by i.p. injection (Table V).

**Table III. Compound 60 produced a time-dependent reduction in the duration of immobility in the mouse FST.**

| Time (post-dose) | Mean duration of immobility (sec) | SEM | n |
|---|---|---|---|
| 0 (saline control) | 128.2 | 7.5 | 18 |
| 0.5 | 96.1 | 11.5 | 8 |
| 1 | 38.8* | 6.1 | 8 |
| 2 | 66.2* | 7.9 | 8 |
| 4 | 66.9* | 13.5 | 8 |
| 6 | 76.8* | 9.0 | 8 |
| 24 | 124.8 | 15.9 | 8 |
| Compound 60 administered as a single dose, 5 mg/kg p.o.<br>* $p < 0.05$ compared to saline control. | | | |

**Table IV. Compound 60 produced a dose-dependent reduction in the duration of immobility in the mouse FST.**

| Dose (mg/kg) | Mean duration of immobility (sec) | SEM | n |
|---|---|---|---|
| 0 (saline control) | 129.7 | 13.3 | 8 |
| 0.625 | 153.3 | 12.0 | 8 |
| 1.25 | 72.3* | 7.1 | 8 |
| 2.5 | 64.5* | 13.4 | 8 |
| 5 | 56.6* | 7.1 | 8 |
| 10 | 68.7* | 13.7 | 6 |

(continued)

| Dose (mg/kg) | Mean duration of immobility (sec) | SEM | n |
|---|---|---|---|
| Imipramine | 53.4* | 18.6 | 8 |
| Compound 60 administered as a single oral dose 60 minutes prior to testing.<br>Imipramine administered as a single dose, 10 mg/kg i.p., 30 minutes prior to testing.<br>* $p < 0.05$ compared to saline control. | | | |

**Table V. Compound 60 produced a dose-dependent reduction in the duration of immobility in the mouse FST.**

| Dose (mg/kg) | Mean duration of immobility (sec) | SEM | n |
|---|---|---|---|
| 0 (saline control) | 137.8 | 6.6 | 22 |
| 0.625 | 139.5 | 12.8 | 10 |
| 1.25 | 85.6* | 11.5 | 10 |
| 2.5 | 68.5* | 7.1 | 16 |
| 5 | 89.6* | 12.0 | 6 |
| 10 | 106.1 | 14.4 | 6 |
| Imipramine | 72.5* | 8.7 | 18 |
| Compound 60 administered as a single i.p. dose. 30 minutes prior to testing.<br>Imipramine administered as a single dose, 10 mg/kg i.p., 30 minutes prior to testing.<br>* $p < 0.05$ compared to saline control. | | | |

Compound 60 produced a dose-dependent antidepressant-like effect in the rat forced-swim test when administered orally 1 hour prior to testing (Table VI). The magnitude of this effect was similar to that produced by imipramine, 10 mg/kg i.p. administered 30 minutes prior to testing.

**Table VI. Compound 60 produced a dose-dependent antidepressant-like effect in the rat FST.**

| Dose (mg/kg) | Mean Change in the Duration of Immobility (Day 2 minus Day 1) (sec) | SEM | n |
|---|---|---|---|
| 0 (saline control) | 47.9 | 11.2 | 14 |
| 1.25 | 29.7 | 11.2 | 11 |
| 2.5 | -24.8* | 13.6 | 9 |
| 5 | -28.8* | 8.5 | 11 |
| Imipramine | -23.3* | 15.2 | 10 |
| Compound 60 administered as a single oral dose 60 minutes prior to testing.<br>Imipramine administered as a single dose, 10 mg/kg i.p., 30 minutes prior to testing.<br>* $p < 0.05$ compared to saline control. | | | |

A single 5-mg/kg oral dose of Compound 60 produced a time-dependent reduction in the duration of immobility in the mouse TST (Table VII), with the peak effect occurring 60 minutes post-dosing. In two independent experiments, Compound 60 produced a dose-dependent reduction in the duration of immobility in the mouse TST when administered as a single oral dose 1 hour prior to testing (Tables VIII and IX). Imipramine, 15 mg/kg i.p., administered 30 minutes prior to testing, was used as a positive control in one of these studies (Table VIII).

**Table VII. Compound 60 produced a time-dependent reduction in the duration of immobility in the mouse TST.**

| Time (post-dose) | Mean duration of immobility (sec) | SEM | n |
|---|---|---|---|
| 0 (saline control) | 154.9 | 10.0 | 13 |
| 0.5 | 84.9* | 12.4 | 6 |
| 1 | 74.7* | 10.5 | 6 |
| 2 | 93.0* | 18.8 | 6 |
| 4 | 109.7 | 9.0 | 6 |
| 6 | 125.5 | 20.8 | 5 |
| 24 | 177.7- | 21.0 | 5 |
| imipramine | 103.6* | 16.9 | 8 |
| Compound 60 administered as a single dose, 5 mg/kg p.o.<br>Imipramine administered as a positive control, 15 mg/kg i.p. 30 minutes prior to testing.<br>* $p < 0.05$ compared to saline control. | | | |

**Table VIII. Compound 60 produced a dose-dependent reduction in the duration of immobility in the mouse TST (experiment #1).**

| Dose (mg/kg) | Duration of Immobility (sec) | SEM | n |
|---|---|---|---|
| 0 (saline control) | 210.1 | 6.0 | 5 |
| 2.5. | 130.7* | 11.2 | 4 |
| 5 | 123.5* | 27.8 | 3 |
| 10 | 47.8* | 16.8 | 6 |
| imipramine | 133.9* | 17.4 | 4 |
| Compound 60 administered as a single oral dose 60 minutes prior to testing.<br>Imipramine administered as a.single dose, 15 mg/kg i.p., 30 minutes prior to testing.<br>* $p < 0.05$ compared to saline control. | | | |

**Table IX. Compound 60 produced a dose-dependent reduction in the duration of immobility in the mouse TST (experiment #2).**

| Dose (mg/kg) | Duration of Immobility (sec) | SEM | n |
|---|---|---|---|
| 0 (saline control) | 149.1 | 15.3 | 8 |
| 0.625 | 143.3 | 13.0 | 6 |
| 1.25 | 125.8 | 9.7 | 8 |
| 2.5 | 69.6* | 17.4 | 8 |
| 5 | 69.5* | 19.8 | 6 |
| 10 | 15.6* | 7.8 | 6 |
| Compound 60 administered as a single oral dose 60 minutes prior to testing.<br>* $p < 0.05$ compared to saline control. | | | |

Example 3: Compound Synthesis

The different compounds described herein can be produced using techniques well known in the art. This example illustrates the use of such techniques to obtain different types of compounds. Other particular compounds described herein can be readily obtained using well-known synthetic techniques, including modifying the procedure described below.

Capillary gas chromatographic and low-resolution electron-impact mass spectral (EI-MS) data were obtained using a Hewlett-Packard (HP) 5890 Series II Gas Chromatograph coupled to an HP 5971 Series Mass Selective Detector [Ultra-2 Ultra Performance Capillary Column (crosslinked 5% PhMe silicone); column length, 25 m; column i.d., 0.20 mm; Helium flow rate, 60 mL/min; injector temp., 250 ˚C; temperature program, 20 ˚C/min from 125 to 325˚C for 10 min, then held constant at 325˚C for 6 min]. Thin-layer chromatography was performed using Analtech Uniplate 250-µm silica gel HF TLC plates. UV light in conjunction with ninhydrin and Dragendorff's spray reagents (Sigma Chemical Co.) were used for detecting compounds on the TLC plates. Reagents used in reactions were purchased from the Aldrich Chemical Co. (Milwaukee, WI), Sigma Chemical Co. (Saint Louis, MO), Fluka Chemical Corp. (Milwaukee, WI), Fisher Scientific (Pittsburgh, PA), TCI America (Portland, OR), or Lancaster Synthesis (Windham, NH).

Reaction Scheme 1: Synthesis of Compound 60

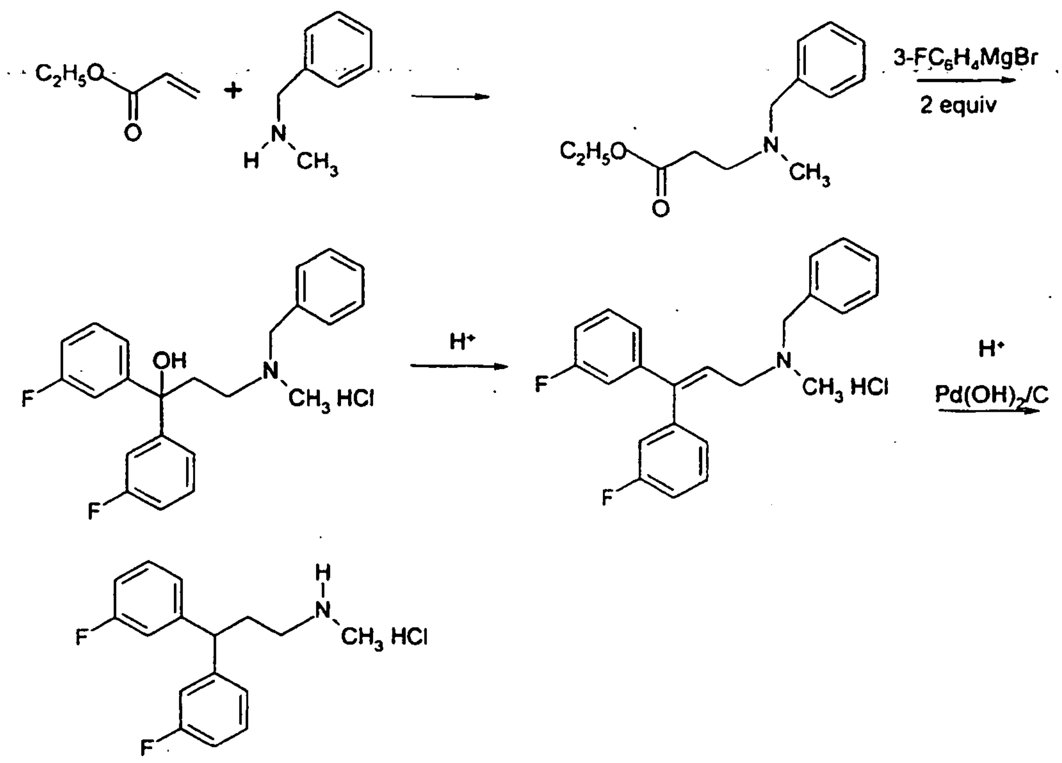


**Compound 60**

Compound 60 was synthesized from commercially available starting materials in the following four-step reaction sequence. The first intermediate in this synthetic route, ethyl-*N*-benzyl-*N*- methyl-3-aminopropionate, was prepared by conjugate addition of *N*-benzylmethylamine to ethyl acrylate. The ester functionality of the first intermediate was then reacted with two equivalents of Grignard reagent (prepared from 1-bromo-3-fluorobenzene) to provide *N*-benzyl-*N*-methyl-3-hydroxy-3-(bis-3-fluorophenyl)-propylamine. The Grignard reaction product was then dehydrated in a mixture of 6 M HCl/acetic acid to yield *N*-benzyl-*N*-methyl-3,3-bis(3-fluorophenyl)-allylamine. Catalytic hydrogenation of this material as its hydrochloride salt in ethanol over Pearlman's catalyst $[Pd(OH_2)/C]$ provided, after recrystallization from ethyl acetate, colorless, needles of Compound 60 hydrochloride.

Ethyl *N*-benzyl-*N*-methyl-3-aminopropionate.

In a 500-mL, 3-necked flask equipped with thermometer, reflux condenser, and a 125-mL addition funnel [charged with ethyl acrylate (88.3 mL, 81.5 g, 0.815 mol)] was placed *N*-benzylmethylamine (100 mL, 94.0 g, 0.776 mol). The ethyl acrylate was added dropwise to the stirring reaction mixture over a period of 80 min. After stirring for 18 h at room temperature, the product was vacuum distilled and the fraction containing product was collected at 78-95 ˚C (0.12-0.25 mm Hg), (138 g, 80% yield): bp 78-95 ˚C (0.12-0.25 mm Hg); TLC, $R_f$ = 0.23 [hexane-EtOAc (5:1)], $R_f$ = 0.57 [MeOH-$CHCl_3$ (100:5)]; GC, $t_R$ = 6.06 min; EI-MS, 221 ($M^+$), 206 (M-$CH_3$), 192 (M-$C_2H_5$), 176 (M-$OC_2H_5$), 144 (M-$C_6H_5$) , 134 [$CH_2N(CH_3)CH_2Ph$], 120 [$N(CH_3)CH_2Ph$], 91 ($C_7H_7$), 77 ($C_6H_5$), 42 ($CH_2CH_2N$); $^1$H NMR (free base, $CDCl_3$) δ 1.25 ppm (t, *J* = 7.1, 3H, $CH_2C\underline{H}_3$), 2.20 (s, 3H, $NC\underline{H}_3$), 2.51 (t, *J* = 7.3, 2H, $COC\underline{H}_2$), 2.74 (t, *J* = 7.2, 2H, $C\underline{H}_2N$), 3.51 (s, 2H, $NC\underline{H}_2Ph$), 4.13 (q, *J* = 7.1, 2H, $OCH_2CH_3$), 7.18-7.35 (m, 5H, ArH); $^{13}$C NMR (free base, $CDCl_3$) δ 15.2 ($CH_2\underline{C}H_3$) , 34.0 ($CO\underline{C}H_2$), 42.9 ($N\underline{C}H_3$), 53.8 ($N\underline{C}H_2$), 61.4 ($O\underline{C}H_2CH_3$), 63.1 ($\underline{C}H_2Ph$), 128.0 (CH), 129.2 (CH), 130.0 (CH), 139.9 (q), 173 .7 (q).

*N*-Benzyl-*N*-methyl-3-hydroxy-3-bis(3-fluorophenyl)propylamine hydrochloride.

In a 5-L,-four-necked, round-bottom flask, under nitrogen, was placed Mg [51.5 g, 2.12 mol, turnings, washed with THF (2 x 300 mL)] and THF (2 L). An addition funnel was charged with 3-bromofluorobenzene (neat, 392.8 g, 2.24 mol). One-twentieth of the bromide was added to the magnesium suspension followed by one crystal of iodine. After initiation of the Grignard reaction the remaining 3-bromofluorobenzene was then added to the refluxing mixture over a period of 50 min. The reaction was refluxed for an additional 45 min. To the refluxing solution of Grignard reagent was added a solution of ethyl *N*-benzyl-*N*-methyl-3-aminopropionate (187.5 g, 0.847 mol) in THF (100 mL) over a period of 20 min. After the ester addition was complete, the reaction was refluxed for 1 h. The reaction was then cooled in an ice bath. Saturated $NH_4Cl$ (aq., 400 mL) and $H_2O$ (400 mL) were added and the mixture was transferred to a separatory funnel. The organic layer was separated and the aqueous layer was extracted once with THF (400 mL). The combined organic layers were washed with satd. NaCl (aq., 2 x 200 mL), dried (anh. $Na_2SO_4$), filtered through paper, and rotary evaporated vacuum to yield 281.6 g (90%) of crude product as an orange, viscous oil. This material (281.6 g, 0.766 mol) was dissolved in acetonitrile (1.4 L). Concentrated hydrochloric acid (65.0 mL, 0.786 mol, 12 M) was added to the stirring filtrate. The crystallizing mixture was then cooled to -20 ˚C for 17 h. The product was collected, washed with cold acetonitrile (800 mL), and dried to provide a white solid, 235.6 g (69% yield from the ester).

For analytical purposes, the hydrochloride salt was further purified by recrystallization from acetonitrile: Mp 194-197 ˚C (uncorr.); TLC, $R_f$ = 0.23 [hexane-EtOAc (5:1)], $R_f$ = 0.85 [MeOH-$CHCl_3$ (100:5)], $R_f$ = 0.72 [MeOH-$CHCl_3$ (100:3)]; GC, $t_R$ = 10.93 min; MS, 367 ($M^+$), 272 (M-$C_6H_4F$), 258 (M-$CH_2Ph$-$H_2O$), 219 [$(C_6H_4F)_2CH$], 148 [$CH_2CH_2N(CH_3)CH_2Ph$], 134 [$CH_2N(CH_3)CH_2Ph$], 91 ($C_7H_7$), 42 ($CH_2CH_2N$); $^1$H NMR (free base, $CDCl_3$) δ 2.18 (s, 3H, $NC\underline{H}_3$), 2.41 (m, 2H, $CHC\underline{H}_2$), 2.58 (m, 2H, $C\underline{H}_2N$), 3.42 (s, 2H, $C\underline{H}_2Ph$), 6.86 (dt, $J_1$ = 8.5, $J_2$ = 1.8, 2H, Ar-$\underline{H}$), 7.18-7.30 (m, 10H, Ar-$\underline{H}$), 8.33 (bs, 1H, $O\underline{H}$); $^{13}$C NMR (free base, $CDCl_3$) δ 35.6 ($CH\underline{C}H_2$), 41.5 ($CH_3$, $N\underline{C}H_3$), 54.3 ($CH_2$, $\underline{C}H_2N$), 62.6 ($CH_2$, $\underline{C}H_2Ph$), 113.1 (d, *J* = 23, CH, Ar-$C_{5,5'}$), 113.5 (d, *J* = 23, CH), 121.2 (d, *J* = 3, CH), 127.5 (CH), 128.5 (CH), 129.2 (CH), 129.5 (CH), 129.6 (CH), 137.0 (q), 150.2 (q), 162.8 (d, *J* = 243, q, Ar-$C_{3,3'}$).

*N*-Benzyl-*N*-methyl-3-bis(3-fluorophenyl)allylamine hydrochloride.

In a 5-L, 3-necked reaction vessel, equipped with an overhead mechanical stirrer, reflux condenser, and thermometer, was placed *N*-benzyl-*N*-methyl-3-hydroxy-3-bis(3-fluorophenyl) propylamine hydrochloride (225.4 g, 0 . 559 mol), 6 M HCl (1392 mL), and glacial HOAc (464 mL). The suspension was heated in a water bath (80-85 ˚C) and stirred for 18 h. After 18 h of heating, the reaction mixture was cooled in an ice/MeOH bath. Ethyl acetate (500 mL) was added to the cooled reaction mixture. NaOH (10 M, 1.7 L) was then added to the cooled mixture over a period of 25 min at such a rate as to keep the temperature below 40 ˚C. The mixture was transferred to a 6-L separatory funnel. The organic layer was separated and the aqueous layer was extracted with ethyl acetate (2 x 500 mL). The combined organic layers were washed with satd. NaCl (aq., 2 x 100 mL), dried ($Na_2SO_4$, 250 g), rotary evaporated, and then dried under vacuum to provide 185.6 g (95% yield) of the free base as a fluid, brownish-colored oil.

The material above was stirred with hexane (1.5 L). The resulting solution was filtered through paper. 4 M HCl in dioxane (146 mL) was added dropwise with stirring to the filtrate over a period of 5 min. The semi-translucent solvent was then decanted away from the light-yellow colored, semisolid precipitate. The crude hydrochloride salt was dissolved in refluxing ethyl acetate (600 mL) and was filtered. The filtrate was then thoroughly cooled in an ice bath, and hexane (110 mL) was slowly added with vigorous stirring. After cooling in an ice bath for 2 h, the entire flask filled with a white crystalline solid. This material was collected on a filter funnel, washed with ice-cold hexane/ethyl acetate [(1:4), 400 mL], and dried to yield 128.7 g, 59.7% of a white solid. On standing the mother liquor precipitated another 14.8 g of an off-white solid. Total yield 128.7 g + 14.8 g = 143.5 (67%) . Mp 141-142 ˚C (uncorr.) ; TLC, $R_f$ = 0.20 [hexane-EtOAC

(5:1)], $R_f$ = 0.75 (MeOH-$CHCl_3$ (100:5)], $R_f$ = 0.49 [MeOH-$CHCl_3$ (100:3)]; GC, $t_R$ = 10.40 min; MS, 349 ($M^+$), 330, 301, 281, 258 (M-$CH_2$Ph), 240, 229 [M-N($CH_3$)$CH_2$Ph], 201, 183, 146, 133, 109, 91 ($CH_2C_6H_5$), 65, 42 ($CH_2NHCH_3$); $^1$H NMR (free base, $CDCl_3$) δ 2.20 ppm (s, 3H, $NC\underline{H}_3$), 3.08 (d, *J* =6. 8, 2H, $C\underline{H}_2$N), 3.47 (d, *J* < 1, 2H, $C\underline{H}_2$Ph), 6.29 (t, *J* = 6.8, 1H, C$\underline{H}$), 6.85-7.04 (m, 6H, ArH), 7.19-7.35 (m, 7H, ArH).

*N*-Methyl-3,3-bis(3-fluorophenyl)propylamine hydrochloride.

*N*-Benzyl-*N*-methyl-3-bis(3-fluorophenyl)allylamine hydrochloride (120.0 g, 0.311 mol) was dissolved in abs. EtOH (1250 mL). $Pd(OH)_2$/charcoal (Fluka®, -20% Pd, 10.0 g) was added. The reaction mixture was stirred under a steady flow of hydrogen gas for 18 h at 25 °C (atmospheric pressure). The mixture was then filtered through Celite®/ fritted glass, the catalyst was washed with EtOH (2 x 50 mL), and the solvent was removed under reduced pressure to yield 95.4 g, 103% of crude product. This material was dissolved in refluxing ethyl acetate (300 mL) with vigorous stirring and filtered. The flask was allowed to stand for 2 h at 25 °C, during which time the hydrochloride salt began to crystallize as needles. The flask was then cooled, the product was collected, washed with ice-cold ethyl acetate (20 mL), and dried to yield 73.7 g, 80% of Compound 60 as a white, crystalline solid.

Mp 129-130 °C; UV/Vis, ε = 2.1 x $10^3$ L·$mol^{-1}$·$cm^{-1}$ (264 nm, EtOH, 25 °C, linear range: 0.05-0.20 mg/mL); TLC, $R_f$ = 0.00 [hexane-EtOAc (5:1)], $R_f$ = 0.07 [MeOH-$CHCl_3$ (100:5)], $R_f$ = 0.19 [MeOH-$CHCl_3$-$NH_4OH$ (100:5:1)]; GC, $t_R$ = 7.45 min; MS, 261 ($M^+$), 229, 215, 201, 183, 164, 150, 138, 122, 101, 83, 75, 57, 42 [$CH_2NHCH_3$]; $^1$H NMR (HCl salt, $CDCl_3$ + 1 gtt MeOD) δ 2.56 ppm (m, 2H, $NC\underline{H}_2$), 2.60 (s, 3H, $NC\underline{H}_3$), 2.85 (t, *J* = 8.0, 2H, $CHC\underline{H}_2$), 4.11 (t, *J* = 8.0, 1H, CH), 6.87-6.98 (m, 4H, ArH), 7.06 (d, *J* = 7.7, 2H, $Ar_{2,2'}$H), 7.25 (dd, $J_1$ = 6, $J_2$ = 8, ArH); $^{13}$C NMR (HCl salt, $CDCl_3$ + 1 gt MeOD) δ 30.9 ($CH_2$, $CH\underline{C}H_2$), 32.7 ($CH_3$, $N\underline{C}H_3$), 47.6 (CH, $\underline{C}HCH_2$), 47.8 ($CH_2$, $\underline{C}H_2$N), 113.9 (*J* = 21, $ArC_{2,2'}$ or $ArC_{4,4'}$), 114.5 (d, *J* = 22, $ArC_{2,2'}$ or $ArC_{4,4'}$), 123.2 (d, *J* = 3, Ar-$C_{6,6'}$), 130.3 (d, *J* = 9, Ar-$C_{5,5'}$), 144.7 (d, *J* = 7, Ar-$C_{1,1'}$), 162.9 (d, *J* = 245, Ar-$C_{3,3'}$); IR: KBr pellet, ($cm^{-1}$) 3436.9, 2963.4, 2778.5, 2453.7, 1610.6, 1589.3, 1487.0, 1445.3, 1246.0, 764.5; solubility: 2 g/mL ($H_2O$), 1 g/mL (EtOH); anal. calcd. for $C_{16}H_{17}NF_2$·HCl (Karl Fischer: 0.26% $H_2O$): C, 64.37; H, 6.11; N, 4.69; found: C, 64.14; H, 6.13; N, 4.69.

Reaction Scheme 2: Synthesis of Compound 119.

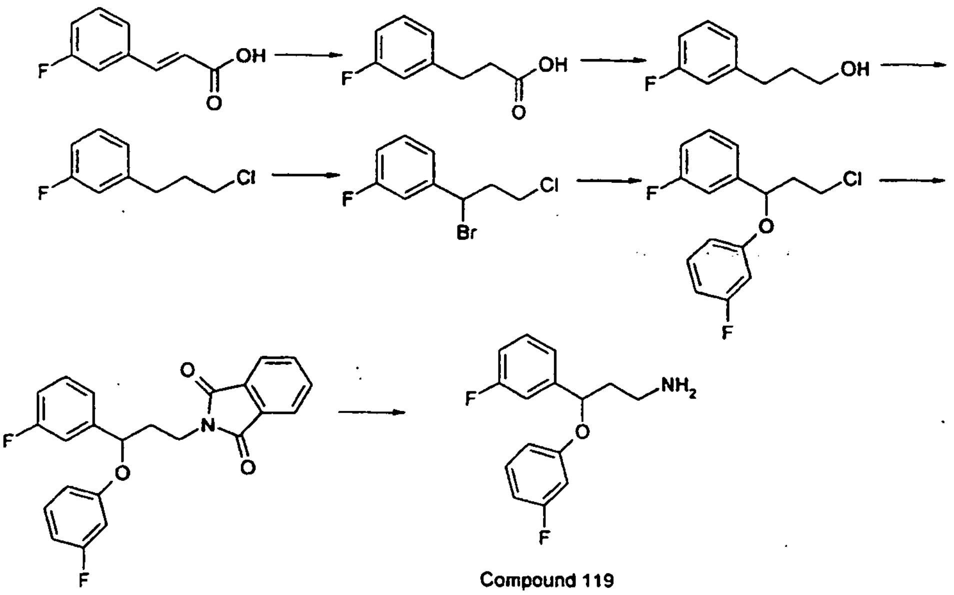

Compound 119

Compound 119 was synthesized in a seven-step reaction w sequence starting from commercially-available trans-3-fluorocinnamic acid. This synthetic route is conceptually similar to that reported in the literature (U.S. Patent 4,313,896 (1982) hereby incorporated by reference herein) for related analogs. However, the three final steps were performed using a significantly different reaction sequence than that reported. The cinnamic acid was reduced and chlorinated in three steps to the corresponding 3-(3-fluorophenyl)propylchloride. This compound was brominated with NBS (N-bromosuccinimide) and the resulting trihalide was then reacted with 3-fluorophenol. The resulting ether was converted to the final product using a Gabriel synthesis.

3-Fluorohydrocinnamic acid.

*Trans*-3-fluorocinnamic acid (25.0 g, 150.4 mmol) was dissolved in abs. EtOH (250 mL) and hydrogenated over 10% Pd/C (2.5 g) in a Parr apparatus at 60 psig, 50 °C, for 1 h (hydrogen uptake: calcd. 245 psig; found 260 psig). The reaction mixture was filtered and evaporated to yield a crystalline product (23.0 g, 89%). GC, $t_R$ = 4.43 min; MS, 168 ($M^+$).

3-(3-Fluorophenyl)-1-propanol.

Under a stream of dry nitrogen, at 0-10 °C, a solution of 3-fluorohydrocinnamic acid (22.0 g, 131 mmol) in THF (100 mL) was added dropwise, over a period of 15 min, to a suspension of $LiAlH_4$ (4.23 g, 111 mmol) in THF (200 .mL). The reaction was heated to reflux for a period of 1 h and then worked-up according to *Fieser & Fieser's Reagents for Organic Synt*hesis (vol. 1, 1967) to provide a white solid (20.1 g, 99%). GC, $t_R$ = 3.74 min; MS, 154 ($M^+$).

3-(3-Fluorophenyl)-1-propylchloride.

A solution of 3-(3-fluorophenyl)-1-propanol (15.0 g, 97.4 mmol) and triphenylphosphine (36.0 g, 137.3. mmol) in $CCl_4$ (150 mL) was refluxed for 19 h. Additional $P(C_6H_5)_3$ (3 x 3.0 g, 3 x 11.4 mmol) was added periodically over a period of 24 h. The resulting precipitate was removed by filtration and the solids were washed with hexane. The filtrate was evaporated under vacuum and the residue was suspended in hexane (200 mL) and then filtered. Evaporation of the filtrate provided 16.0 g (95.1%) of crude product which was purified by silica gel flash chromatography, elution with hexane, to provide 14.7 g (87%) of a colorless liquid. GC, $t_R$ = 3.63 min; MS, 172/174 ($M^+$).

3-Bromo-3-(3-fluorophenyl)-1-propylchloride.

A solution of the above chloride (12.0 g, 69.5 mmol), *N*-bromosuccinimide (17.3 g, 97.2 mmol), and dibenzoyl peroxide (0.06 g) in $CCl_4$ (75 mL) was refluxed for 1 h. The reaction mixture was then cooled in an ice bath, filtered, and the solids were washed with hexane. The filtrate was evaporated to provide 17.9 g (100%) of product. GC, $t_R$ = 5.21 min; MS, 251/253 ($M^+$).

3-(3-Fluorophenoxy)-3-(3-fluorophenyl)-1-propylchloride.

A mixture of 3-bromo-3-(3-fluorophenyl)-1-propylchloride (4.0 g, 15.9 mmol), 3-fluorophenol (1.98 g, 17.7 mmol), and $K_2CO_3$ (2.65 g, 19.2 mmol) suspended in acetone (80 mL) was refluxed for 15 h. The volatiles were then removed under vacuum and the resulting residue was suspended in a mixture of hexane (200 mL) and NaOH (0.1 M, 100 mL). The layers were separated and the organic layer washed, 0.1 M NaOH (100 mL) and $H_2O$ (100 mL), dried (anh. $Na_2SO_4$), and evaporated *in vacuuo.* The resulting residue was chromatographed on silica gel, elution with hexane followed by hexane/EtOAc [100:1] then [40:1] to provide 1.64 g (37%) of product as a colorless oil. GC, $t_R$ = 7.28 min; MS, 282/283 ($M^+$); TLC $r_f$ = 0.3, hexane/EtOAc [40:1].

*N*-Phthaloyl-3-(3-fluorophenoxy)-3-(3-fluorophenyl)-1-propylamine.

A solution of 3-(3-fluorophenoxy) -3- (3-fluorophenyl) -1-propylchloride (1.52 g, 5.38 mmol) and potassium phthalate (1.20 g, 6.48 mmol) was heated to 90 °C in DMF (30 mL) for a period of 2 h in a nitrogen atmosphere. The reaction mixture was then cooled and poured into $H_2O$ (100 mL). The resulting solution was extracted with $Et_2O$ (2 x 100 mL). The organic extract was washed, satd. aq. NaCl (100 mL) and $H_2O$ (2 x 100 mL), dried (anh. $Na_2SO_4$), and evaporated under vacuum to provide 2.17 g of crude product. The material was chromatographed on silica gel, elution with hexane/EtOAc [40:1] and then [20:1] to provide after evaporation 1.81 g (86%) of product as a glass.

3-(3-Fluorophenoxy)-3-(3-fluorophenyl)-1-propylamine.

A solution of *N*-phthaloyl-3-(3-fluorophenoxy)-3-(3-fluorophenyl)-1-propylamine (1.74 g, 4.42 mmol) and anh. hydrazine (1.43 g, 44.6 mmol) in abs. EtOH (30 mL) was refluxed for 1 h. The reaction was cooled and evaporated under vacuum. The resulting material was suspended in $Et_2O$ (75 mL) and washed with 0.2 M NaOH (2 x 25 mL). The organic layer was dried (anh. $Na_2SO_4$) and evaporated under vacuum to provide 1.04 g (89.3%) which was purified by reverse-phase chromatography [Vydac Prep. C18; 264 nm; 50 mL/min; gradient elution ACN/0.1% HCl aq., 10%-50% over 20 min; $r_t$ = 17.4 min], to yield 0.89 g (67%) of Compound 119 as a hygroscopic hydrochloride salt.

In a similar manner, Compound 236 can be synthesized using the previously described synthetic protocol with

the following substitution: 4-trifluorophenol material substituted in Reaction scheme 2 for 3-fluorophenol.

Reaction Scheme 3: Synthesis of Compound 185

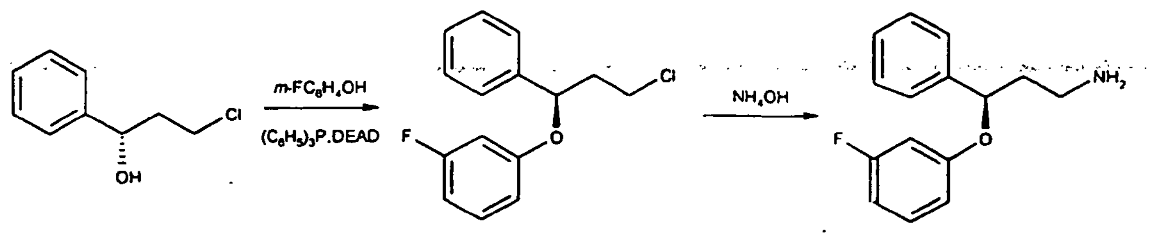


Compound 185

Compound 185 was prepared following a similar procedure for the chiral synthesis of fluoxetine (Brown, H.C. *et al., J. org. Chem*. 53(13), 2916-2920(1988)).

(*R*)-3-(3-fluorophenoxy)-3-phenylpropylchloride.

A solution of (*S*)-(-)3-chloro-1-phenyl-1-propanol (4.00 g, 23.4 mmol), 3-fluorophenol (2.63 g, 23.4 mmol), and diethyl azodicarboxylate (4.00 g, 23.4 mmol) was dissolved in THF (200 mL). The mixture was cooled to 0 ˚C and triphenylphosphine (6.77 g, 25.8 mmol, 1.1 equiv) was added slowly over 10 min. The reaction mixture was then stirred at room temperature for 18 h. The THF was subsequently evaporated under vacuum to afford a gel which was washed with pentane (3 x 50 mL). The pentane washings were filtered and the filtrate was evaporated under vacuum to give a clear oil. This oil was dissolved in diethyl ether (150 mL) and washed with 1% HCl/satd. aq. NaCl (25 mL), 0.1 M NaOH/ satd. aq. NaCl (2 x 25 mL), and finally $H_2O$ (2 x 25 mL). The organic layer was then dried (anh. $Na_2SO_4$), filtered, and evaporated to dryness under vacuum to give an oil. The crude product was chromatographed on silica gel, elution with 40:1 hexane-EtOAc, to provide 971 mg (15.7%) of product as a colorless oil.

(*R*)-3-(3-Fluorophenoxy)-3-phenylpropylamine.

A solution of (*R*)-3-(3-fluorophenoxy)-3-phenylpropyl chloride (0.971 g, 3.96 mmol), conc. $NH_4OH$ (30 mL), and EtOH (20 mL) was shaken at 90 ˚C on a Parr® apparatus (50-90 psig) for 18 h. The mixture was then evaporated under vacuum and the residue was dissolved in $Et_2O$ (100 mL) and washed with $H_2O$ (2 x 25 mL). The organic layer was dried (anh. $Na_2SO_4$), filtered, and evaporated under vacuum to provide an oil. This material was then dissolved in EtOAc (50 mL) and filtered. A solution of maleic acid (0-272 g, 2.6 mmol, 0.93 equiv) dissolved in hot EtOAc (5 mL) was added to precipitate Compound 185 as its white solid maleate salt (519 mg, 53.5%): TLC $R_f$ 0.25 (1% MeOH/$CHCl_3$) ; GC, $t_r$ 7.37 min; EI-MS, *m*/*z* 245 (M+).

Reaction Scheme 4: Synthesis of Compound 156

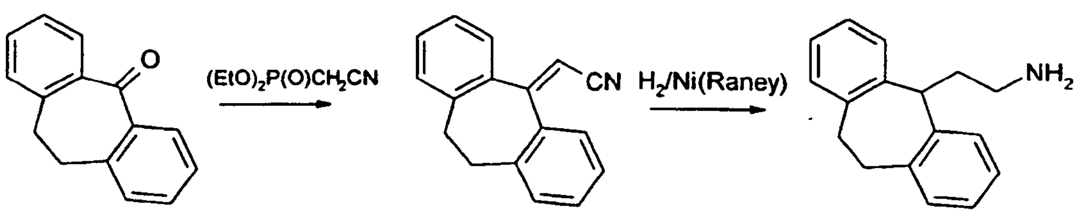


Compound 156

5-Cyanomethylidino-10,11-dihydrodibenzo[*a,d*]cycloheptene.

To a solution of diethyl cyanomethylphosphonate (9.66 g, 54.5 mmol) in dry *N,N*-dimethylformamide (DMF, 40 mL) was added NaH (60% dispersion, 2.20 g, 55.0 mmol) over a period of 2 min. The reaction was stirred for 10 min and then a solution of dibenzosuberone (10.3 g, 49.6 mmol) in dry DMF (10 mL) was added over a period of 2 min. The reaction was stirred at 80 ˚C for 4 h under $N_2$. Water (200 mL) was added and the reaction mixture was extracted with $Et_2O$ (2 x 100 mL). The combined organic layers were rotary evaporated to less than 50 mL. The resulting crystals were collected and washed with cold $Et_2O$ (2 x 50 mL) to yield 7.48 g (65.3%) of product.

5-(2-Aminoethyl)-5*H*-10,11-dihydrodibenzo[*a,d*]cycloheptene hydrochloride.

The conjugated nitrile from above was dissolved in EtOH (100 mL). 1 M NaOH (10 mL) and Raney® nickel (aq. suspension, 0.50 g) were added. The reaction mixture was shaken under 60 psig $H_2$ at 50 ˚C for 22 h, and was then filtered through Celite®. The filtrate was rotary evaporated and the residue was dissolved in $Et_2O$ (100 mL) and washed with satd. aq. NaCl (50 mL) and $H_2O$ (50 mL). The $Et_2O$ layer was dried (anh. $Na_2SO_4$) and rotary evaporated to give the crude product (850 mg) as a colorless oil. This oil was dissolved in EtOAc (5 mL) and filtered. 1.0 M HCl in $Et_2O$ (5 mL) was added to the filtrate and a white, crystalline solid precipitated. This material was recrystallized from EtOH (5 mL)/$Et_2O$ (12 mL) to yield 600 mg (50.7%) of Compound 156 as a white powder.

Synthesis of Compound 65

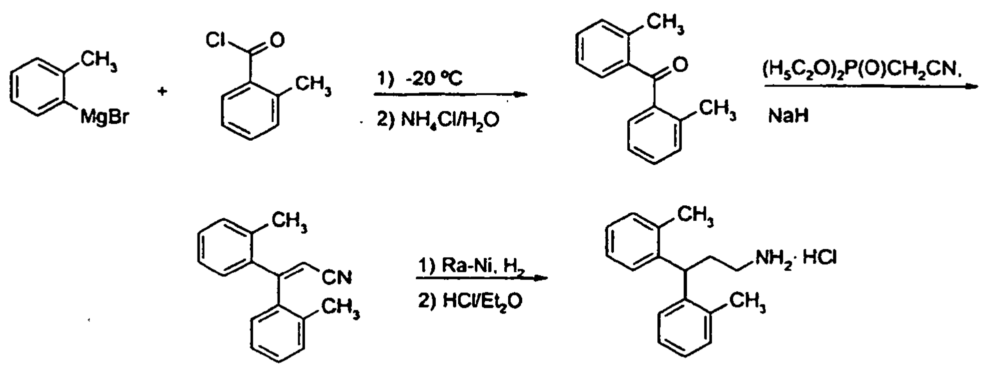


o-Toluoyl chloride (31.0 g, 201 mmol) in diethyl ether (50 mL) was added over a period of 10 min to o-tolylmagnesium bromide (2.0 M in diethyl ether; 105 mL, 210 mmol) cooled to -20 ˚C in a methanol/ice bath. The cold bath was then removed. A voluminous amount of precipitate formed in the reaction mixture. After stirring 10 min, satd. aq. $NH_4Cl$ (200 mL) was carefully added over a period of 8 min. The organic layer was separated. The aqueous layer was extracted with diethyl ether (50 mL). The combined organic layers were washed with $H_2O$ (2 x 50 mL), dried (anhyd. $Na_2SO_4$), and rotary evaporated to yield 41.6 g (98.7%) of product.

Sodium hydride (60% in mineral oil; 3.2 g, 1.9 g NaH, 80 mmol) was added to a solution of diethyl cyanomethylphosphonate (14.2 g, 80.2 mmol) in DMF (75 mL) over a period of 2 min. The reaction mixture was stirred at 80 ˚C for 30 min. 2,2'-dimethylbenzophenone (15.2 g, 72.3 mmol) in DMF (15 mL) was then added to the reaction mixture over a period of 2 min. The reaction mixture was stirred at 80 ˚C. At 27 h $H_2O$ (300 mL) was added to the reaction mixture. This mixture was extracted with diethyl ether (2 x 100 mL). The combined organic layers were washed with $H_2O$ (2 x 100 mL), dried (anh. $Na_2SO_4$), and rotary evaporated to give 15.96 g. The resulting oil was flash chromatographed (step gradient: hexanes; 40:1 hex/EtOAc; 20:1 hex/EtOAc) through flash silica gel (250 x 50 mm) to yield 6.33 g of product.

This material (6.33 g) was dissolved in EtOH (300 mL). Raney nickel (Fluka®; ~50% slurry in $H_2O$; 3.2 g in 1 M NaOH) was added to the filtrate. The reaction mixture was shaken under 60 psig $H_2$ at 60 ˚C for 18 h. The reaction mixture was then filtered through paper and the filtrate was rotary evaporated. This material was dissolved in diethyl ether (100 mL) and washed with $H_2O$ (2 x 50 mL). The organic layer was dried (anh. $Na_2SO_4$) and rotary evaporated to yield 5.61 g of product. This oil was dissolved in EtOAc (60 mL). The HCl salt of the amine was formed by adding 1.0 M HCl in diethyl ether (30 mL). More diethyl ether (30 mL) was added to the mixture. The precipitate was collected and washed with diethyl ether (2 x 50 mL). The resulting white solid was recrystallized from ETOH (60 mL) /diethyl ether (120 mL) to yield 3.32 g of the crystalline hydrochloride salt.

Synthesis of Compound 111

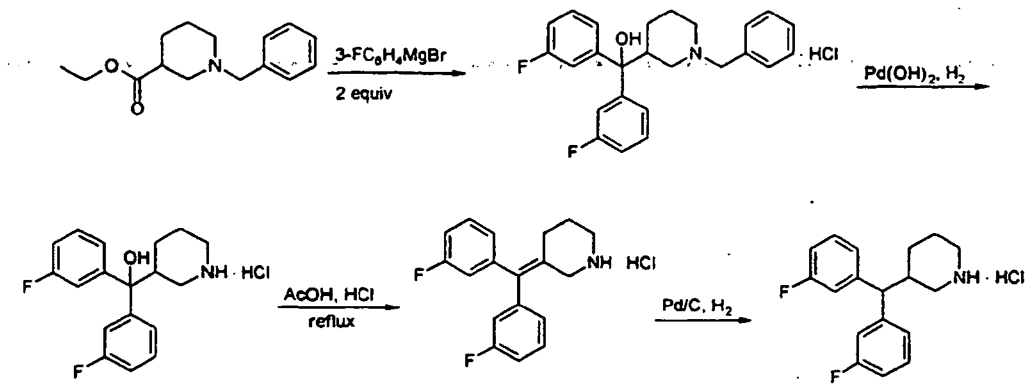


3-Bromofluorobenzene (15.00 g) and magnesium turnings (1.95 g) were dissolved in anh. THF (150 mL). The reaction mixture was refluxed under nitrogen for 30 min. *racemic*-Ethyl *N*-benzylnipecotate (8.00 g, 32.3 mmol) in anh. THF (10 mL) was added over a period of 1 min. The reaction mixture was refluxed for 1.5 h and was then allowed to cool. Satd. aq. $NH_4CL$ (50 mL) was added and the mixture was transferred to a separatory funnel containing satd. aq. NaCl (250 mL) and diethyl ether (150 mL). The organic layer was separated, washed with $H_2O$ (50 mL), dried (anh. $Na_2SO_4$), and rotary evaporated to give 13.0 g of amine. This material was flash chromatographed (gradient: hexanes, 20:1 hex/EtOAc, 9:1 hex/EtOAc, 4:1 hex/EtOAc) through flash silica gel. The fractions containing only product were combined. To this solution was added 1.0 M HCl in diethyl ether (40 mL) This solution was then rotary evaporated and dried under high vacuum to yield 10.93 g (78.6%) of product.

The intermediate prepared above (10.73 g, 24.96 mmol) was dissolved in EtOH (200 mL). Pearlman's catalyst (Fluka®; ~20% Pd; 2.15 g) was added. The reaction mixture was shaken under 50 psig $H_2$ at 50°C for 2 h. The reaction mixture was then filtered through Celite, and the filtrate was rotary evaporated to yield 8.15 g (96.1%) of product. The intermediate prepared above (7.64 g, 22.5 mmol) was dissolved in glacial acetic acid (75 mL). Conc. aq. HCl (75 mL) was added. The reaction mixture was refluxed under $N_2$ flow for 5 h. The reaction mixture was then rotary evaporated to give 7.03 g (97.2%) of product. This solid was dissolved in refluxing ethanol (70 mL). This solution was filtered, more diethyl ether (210 mL) was added, and the resulting crystals were filtered, washed with diethyl ether (2 x 50 mL), and dried under high vacuum to yield 6.27 g (86.7%) of product. The intermediate alkene prepared above (5.66 g, 17.6 mmol) was dissolved in ethanol (200 mL). Palladium on charcoal (~10% Pd; 1.13 g) in $H_2O$ (5 mL) was added. The reaction mixture was shaken under 60 psig $H_2$ at 60 °C for 16.5 h. The reaction mixture was filtered through Celite, and the filtrate was rotary evaporated to give 5.54 g of product. This material was crystallized from ethanol (10 mL)/diethyl ether (40 mL). The crystals were filtered, washed with diethyl ether (2 x 50 mL), and dried under high vacuum to yield 4.82 g (84.6%) of product.

Synthesis of Compound 216 and Compound 217

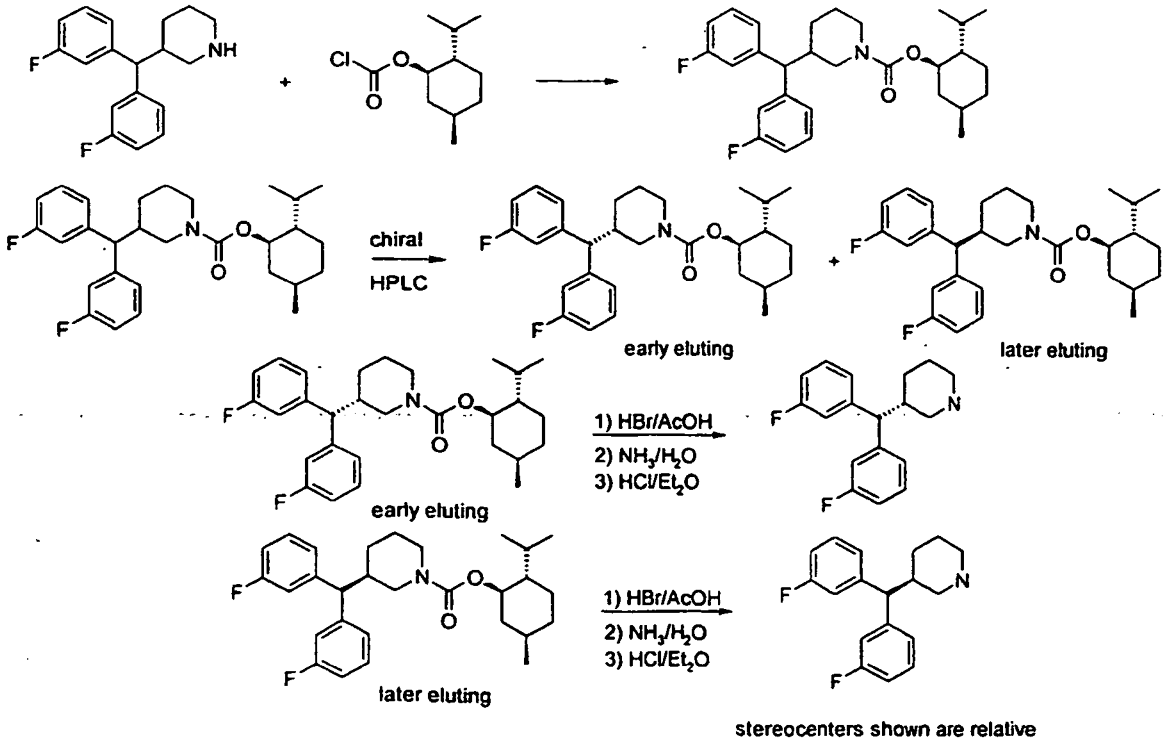


(-)-Menthyl chloroformate (1.57- mL, 1.60 g, 7.32 mmol) was added to a solution of *racemic*-3-[*bis*(3-fluorophenyl)methyl]piperidine (2.10 g, 7.31 mmol) and triethylamine (3.1 mL, 22 mmol) dissolved in $CH_2Cl_2$ (40 mL). After 5 min the reaction solution was rotary evaporated. This material was then flash chromatographed (9:1 hex/EtOAc) through flash silica gel to yield 2.90 g (84.5%) of product.

The resulting oil (2.90 g) was separated on chiral stationary-phase HPLC (Chiralcel OD; column: 20 x 250 mm; eluent: 19:1 hexanes/isopropanol; flow rate: 10 mL/min; load: 1 mL of 50 mg/mL; detector: 259 nm). Rotary evaporation yielded 1.28 g (44.1%) of the early diastereomer and 1.24 g (42.8%) of the late for a total yield of 86.9%. Analytical HPLC showed >99.5% purity for the early diastereomer and >99.0% purity for the late diastereomer.

The early eluting diastereoisomer (959 mg, 2.04 mmol) was dissolved in 30% HBr in acetic acid (20 mL). The reaction solution was stirred at 80 ˚C for 14 h. Ice (~20 g) was added to the reaction mixture followed by $H_2O$ (20 mL), conc. aq. $NH_4OH$ (29%, 10 mL), and satd. aq. $NaHCO_3$) (10 mL). This mixture was extracted with $CHCl_3$ (2 x 30 mL). The combined organic layers were washed with satd. aq. $NaHCO_3$ (30 mL), dried (anh. $Na_2SO_4$), and rotary evaporated to give 921 mg of crude product. The resulting oil was flash chromatographed. (gradient elution: $CHCl_3$, 1:10 MeOH/$CHCl_3$, 0.03:1:10 $NH_3$/MeOH/$CHCl_3$) through flash silica gel. Fractions containing product were combined and rotary evaporated to yield 610 mg (104%) of an oil. This material was dissolved in $CHCl_3$ (10 mL) and washed with 1 M NaOH (10 mL). The organic layer was dried (anh. $MgSO_4$) and rotary evaporated to give 421 mg (71.7%) of product. This material was dissolved in EtOAc (2.0 mL). A solution of 1.0 M HCl in diethyl ether (3.0 mL) was added. Diethyl ether (1.0 mL) was added, and the crystallizing solution was heated to clearness and then allowed to stand. The resulting crystals were washed with diethyl ether (2 x 5 mL) and dried under high vacuum to yield 415 mg (62.8%) of product as a crystalline solid.

The later eluting diastereoisomer (1.24 g, 2.64 mmol) was dissolved in 30% HBr in acetic acid (20 mL). The reaction solution was stirred at 80 ˚C for 16 h. Ice (~25 g) was added to the reaction mixture followed by $H_2O$ (25 mL), conc. aq $NH_3$ (29%, 10 mL), and satd. aq. $NaHCO_3$ (10 mL). This mixture was extracted with $CHCl_3$ (2 x 30 mL). The combined organic layers were washed with 1 M NaOH (2 x 30 mL), dried (anh. $Na_2SO_4$), and rotary evaporated to give 1.07 g. This material was flash chromatographed (gradient elution: $CHCl_3$, 0.03:1:10 $NH_3$/MeOH/$CHCl_3$) through flash silica gel to yield 625 mg (82.4%) of amine. This material was dissolved in EtOAc (3.0 mL). A solution of 1.0 M HCl in diethyl ether (3.0 mL) was added. Diethyl ether (3.0 mL) was added, and the crystallizing solution was heated to clearness and then allowed to stand. The supernatant was decanted and the resultant crystals were washed with diethyl ether (2

x 10 mL) and dried under high vacuum to yield 565 mg (66.1%) of product as a crystalline solid.

## Synthesis of Compound 223 (Flunamine)

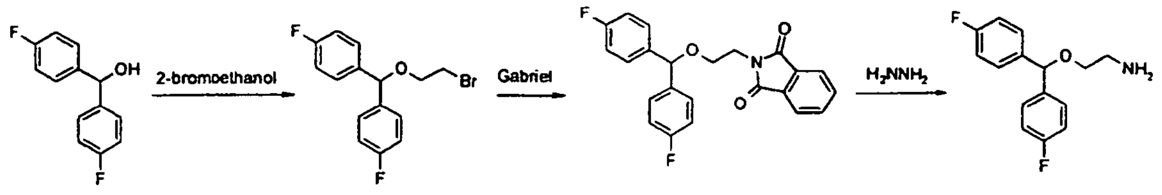


synthesis references: *Collect. Czech. Chem. Commun.* 49(11), 2649-2660 (1984) , *J. Med. Chem.* 35(22), 4238-4248(1992).

A solution of 4,4'-difluorodiphenylmethanol (12.5 g, 56.9 mmol), 2-bromoethanol (7. 11 g, 56.9 mmol), conc. $H_2SO_4$ (2 drops) in toluene (100 mL) was refluxed for 1.5 h. The reaction was then evaporated under vacuum. The residue was dissolved in diethyl ether (100 mL), washed with $H_2O$ (2 x 25 mL), dried (anh. $Na_2SO_4$), and evaporated under vacuum to provide 18. 62 g, 100% yield of crude bromide.

A solution of the bromide prepared above (18.62 g, 56.94 mmol) and potassium phthalimide (10.55 g, 56.94 mmol) in DMF (100 mL) was heated in an oil bath to 120 °C for 10 min. The reaction was then cooled to 25 °C, diethyl ether (400 mL) was added, and the mixture was washed with brine (5 x 100 mL), dried (anh. $Na_2SO_4$), and then one-half of the solvent was evaporated under reduced pressure. The product crystallized from the evaporating solvent, was collected on a funnel, and dried to provide 13.0 g, 58% yield of colorless crystals.

To a suspension of the phthalimide prepared above (13.0. g, 33.1 mmol) in methanol (100 mL) was added anh. hydrazine (5.2 mL, 5.3 g, 165 mmol). The reaction was heated at reflux for 30 min. The volatiles were then removed under vacuum. The residue was dissolved in a mixture of diethyl ether (300 mL), 1 M NaOH (50 mL), and $H_2O$ (200 mL). The layers were separated and the organic layer was washed with 1 M NaOH (50 mL) and $H_2O$ (50 mL), dried (anh. $Na_2SO_4$), and evaporated to give 6.7 g of an oil. This oil, dissolved in EtOAc (20 mL), was added to a solution of maleic acid (2.98 g, 25.7 mmol) in hot EtOAc (40 mL) . The maleate salt crystallized, was collected, washed with diethyl ether (20 mL), and dried under vacuum to provide 8.34 g colorless crystals. GC/MS rt 6.93 min; EI-MS, m/z 264; TLC, 5% MeOH/$CHCl_3$, Rf 0.25.

## Synthesis of Compound 218

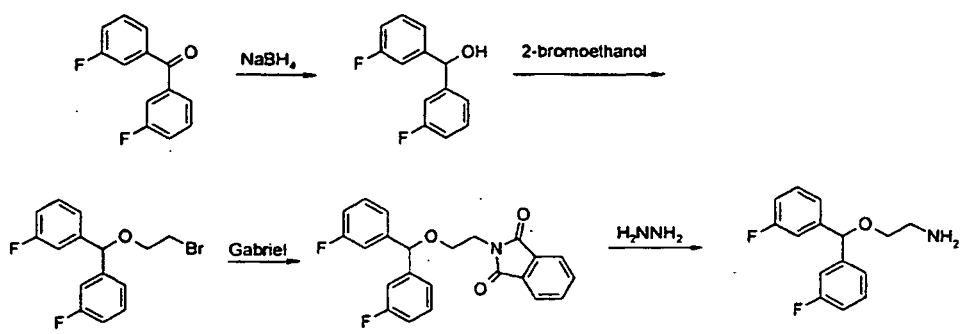


To a solution of 3,3'-difluorobenzophenone (15.0 g, 68.7 mmol) in ethanol (50 mL) was added sodium borohydride (2.86 g, 75.6 mmol). The reaction mixture was then heated to reflux for 15 min. The reaction was then cooled and the solvent evaporated under vacuum. The residue was dissolved in diethyl ether (100 mL), washed with $H_2O$ (3 x 50 mL), dried (anh. $Na_2SO_4$), and evaporated to provide 11.61 g, 76.8% yield of product as an oil: TLC hex/EtOAc [10: 1], Rf = 0.4.

A solution of 3,3'-difluorobenzhydrol (11.61 g, 52.8 mmol), 2-bromoethanol (7.26 g, 58.1 mmol), and conc. $H_2SO_4$ (2-drops) in toluene (100 mL) was refluxed for 1.3 h using a Dean-Starke trap to remove water. The reaction

was then evaporated under vacuum. The residue was dissolved in diethyl ether (100 mL), washed with $H_2O$ (2 x 25 mL), dried (anh. $Na_2SO_4$), and evaporated under vacuum to provide 17.28 g, 100% yield of crude bromide as an oil.

A solution of the crude bromide prepared above (17.28 g, 52.8 mmol) and potassium phthalimide (10.77 g, 58.13 mmol) in DMF (100 mL) was heated in an oil bath to 120 ˚C for 60 min. The reaction was then cooled to 25 ˚C, diethyl ether (400 mL) was added, and the mixture was washed with brine (100 mL), 1 M NaOH (100 mL), then brine again (3 x 100 mL), dried (anh. $Na_2SO_4$), and then most of the solvent was evaporated under reduced pressure. The product crystallized, was collected on a funnel, washed twice with hexane/ether [1:1], and dried to provide 12.51 g, 60.5% yield of colorless crystals. The mother liquor contained 3.72 g (17.9%) of product, for a total yield of 78.2%.

To a suspension of the phthalimide prepared above (12.5 g, 31.8 mmol) in methanol (100 mL) was added anh. hydrazine (5.0 mL, 5.1 g, 159 mmol). The reaction was heated to reflux for 30 min. The volatiles were then removed under vacuum. The residue was dissolved in a mixture of diethyl ether (300 mL), 1 M NaOH (50 mL), and $H_2O$ (200 mL). The layers were separated and the organic layer was washed with 1 M NaOH (50 mL) and the $H_2O$ (3 x 50 mL), dried (anh. $Na_2SO_4$), and evaporated to give 7.58 g, 95% yield of free base as a colorless oil. This material, dissolved in ethyl acetate (20 mL), was added to a solution of maleic acid 3.5 g, 30.2 mmol in hot ethyl acetate (40 mL). The maleate salt crystallized, was collected, washed with ether (20 mL), and dried under vacuum to provide 9.64 g, 80% (overall yield) colorless crystals. GC/MS $t_R$ 6.49 min, m/z 264; TLC 5% MeOH/$CHCl_3$, rf 0.25.

## Synthesis of Compound 219

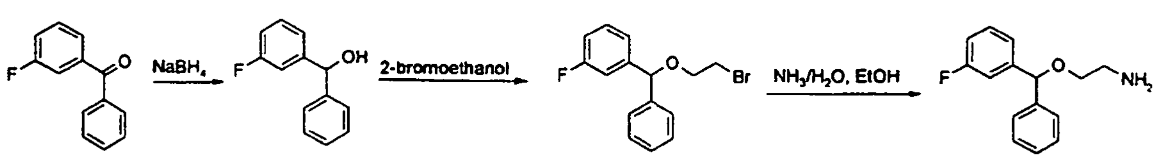


To a solution of 3-fluorobenzophenone (6.67 g, 33.3 mmol) in ethanol (25 mL) was added sodium borohydride (1.40 g, 37.0 mmol). The reaction was exothermic. After stirring 5 min the reaction mixture was rotary evaporated. The resulting material was dissolved in diethyl ether (50 mL), washed with $H_2O$ (2 x 25 mL), dried (anhyd $Na_2SO_4$), and rotary evaporated. The resultant oil was flash chromatographed (gradient elution: hexane, 9:1 hex/EtOAc, 4:1 hex/EtOAc) through flash silica gel to provide 5.97 g (88.6%) of product.

A solution of 3-fluorobenzhydrol (5.97 g, 29.5 mmol), 2-bromoethanol (2.30 mL, 32.4 mmol), and conc. $H_2SO_4$ (2 drops) in toluene (100 mL) was refluxed for 1 h using a Dean-Stark trap to remove $H_2O$. The reaction mixture was washed with $H_2O$ (25 mL) and satd. aq. NaCl (25 mL), dried (anhyd. $Na_2SO_4$), and rotary evaporated (azeotroped with methanol) to provide 7.37 g of crude product. This material was chromatographed (9:1 hex/EtOAc) by MPLC to yield 6.01 g (65.8%) of purified product.

The bromide prepared above (4.90 g, 15.8 mmol) was dissolved in ethanol (100 mL). Concentrated ammonium hydroxide (29% in $H_2O$; 100 mL) was added. In a 500-mL Parr® apparatus, the reaction mixture was shaken at 90 ˚C and 65 psig for 4 h. The reaction mixture was diluted with $H_2O$ (200 mL) and extracted with EtOAc (200 mL). The organic layer was dried (anh. $Na_2SO_4$) and rotary evaporated (azeotroped with benzene) to yield 3.42 g (88.0%) of crude product. This material was dissolved in EtOAc (15 mL) and filtered. The filter was then rinsed with more EtOAc (5 mL). A solution of maleic acid (1.38 g) in EtOAc (20 mL) was added to the combined amine solutions. The acid container was then rinsed with more EtOAc (10 mL). After standing, the resultant crystals were filtered, washed with EtOAc (2 x 20 mL), and dried under high vacuum to provide 3.58 g (62.5%) of maleate salt GC/MS rt 7.12 min, m/z 239; TLC [1:10] MeOH/$CHCl_3$, rf 0.3.

Synthesis of Compound 220

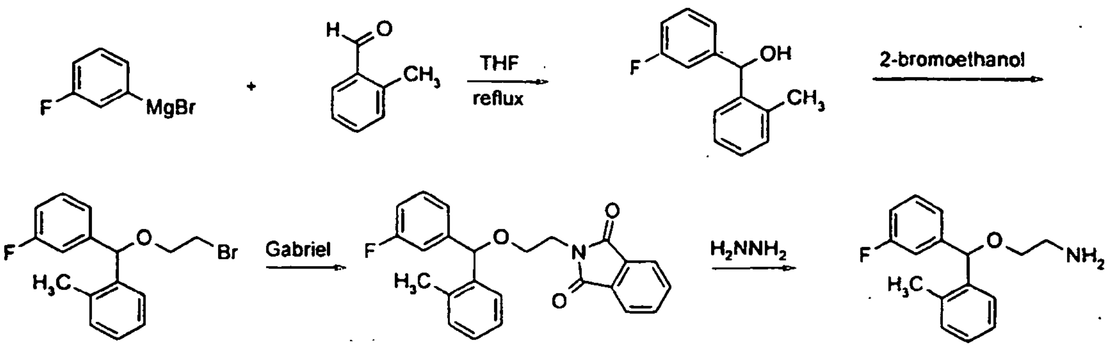


Magnesium turnings (1.22 g, 50.2 mmol) were washed with anh. THF (2 x 50 mL). Anh. THF (100 mL) was then added to the magnesium along with a one crystal of iodine and 10% of a solution of 3-bromofluorobenzene (9.61 g, 54.9 mmol) in anh. THF (50 mL). Under argon flow, the reaction mixture was heated to reflux at which time the reaction initiated. The remaining 3-bromofluorobenzene solution was added over a period of 15 min. The reaction mixture was refluxed for 30 min. While still refluxing, o-tolualdehyde (5.48 g, 45.6 mmol) in anh. THF (25 mL) was added over a period of 5 min. The reaction mixture was refluxed for 15 min and was then quenched with satd. aq. $NH_4Cl$ (50 mL). The aqueous layer was separated. The organic layer was washed with satd. aq. NaCl (2 x 50 mL), dried (anh. $Na_2SO_4$), rotary evaporated (90 °C), and put under high vacuum for 1 h. This provided 9.06 g (91.9%) of product.

A solution of the substituted benzhydrol prepared above (9.06 g, 41.9 mmol) and 2-bromoethanol (5.76 g, 46.1 mmol) in toluene (100 mL) with $H_2SO_4$ (2 drops) was refluxed for 1 h using a Dean-Stark trap for 15 min to remove $H_2O$. The reaction mixture was then rotary evaporated. The residue was dissolved in diethyl ether (100 mL), washed with $H_2O$ (2 x 25 mL), dried (anhyd $Na_2SO_4$), and rotary evaporated to provide 9.71 g (71.7%) of product.

A solution of the above bromide (9.71 g, 30.1 mmol) and potassium -phthalimide (5.23 g, 33.1 mmol) in DMF (100 mL) was heated in an oil bath at 120 °C for 60 min. The reaction was then cooled to 25 °C, diethyl ether (400 mL) was added, and the mixture was washed with $H_2O$ (100 mL), 1 M NaOH (100 mL), then $H_2O$ (3 x 100 mL), dried (anh. $Na_2SO_4$), and then the solvent was evaporated under reduced pressure. The crude material was chromatographed on silica gel (elution with hex/EtOAc [4:1]) to provide 7.96 g, 68.0% of an oil which crystallized on standing.

To a suspension of the above phthalimide (7.96 g, 20.5 mmol) in methanol (100 mL) was added anh. hydrazine (3.2 mL, 3.3 g, 102 mmol). The reaction was heated to reflux for 90 min. The volatiles were then removed under vacuum. The residue was dissolved in a mixture of diethyl ether (300 mL), 1 M NaOH (50 mL), and $H_2O$ (200 mL). The layers were separated and the organic was washed with 1 M NaOH (50 mL) and then $H_2O$ (3 x 50 mL), dried (anh. $Na_2SO_4$), and evaporated to give 4.47 g, 84.1% yield of an oil.

This material, dissolved in EtOAc (10 mL), was added to a solution of maleic acid (2.00 g, 17.3 mmol) in hot EtOAc (20 mL). The maleate salt crystallized, was collected, washed with diethyl ether (20 mL), and dried under vacuum to provide 5.29 g, 68.8% (overall yield) colorless crystals. GC/MS: m/z = 259, rt = 6.95 min; TLC 5% MeOH/$CHCl_3$, rf =0.23.

Synthesis of Compound 221

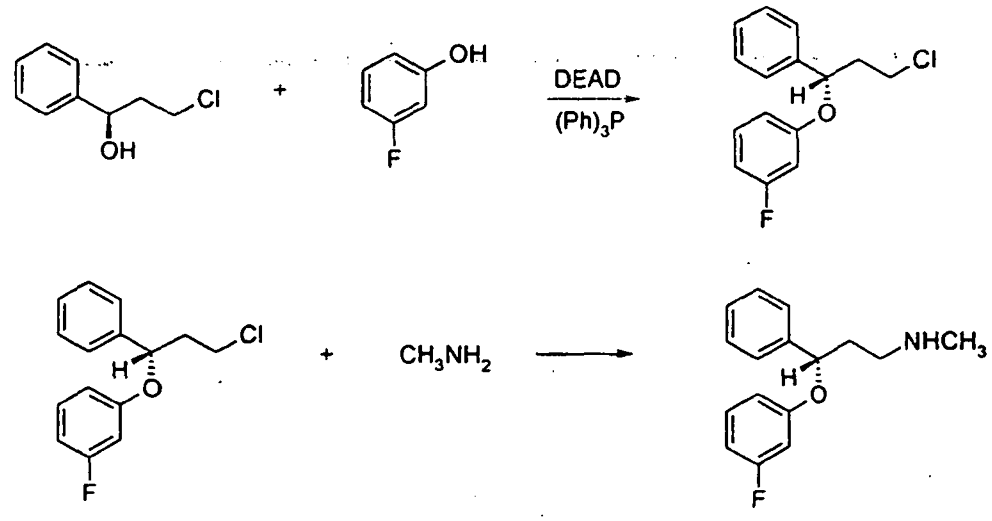


Reference: Mitsunobu, et al., *Synthesis* **1981**, 1-28.

In a 500-mL round bottom flask, triphenylphosphine (19.93 g, 76.18 mmol) was dissolved in THF (50 mL). To this solution was added (*R*)-(+)-chloro-1-phenyl-1-propanol (10.00 g, 58.6 mmol) and 3-fluorophenol (6.57 g, 58.6 mmol) and stirred in an ice bath for 10 min. Finally diethyl azodicarboxylate (13.27 g, 76.18 mmol) was added dropwise over 5 min. The reaction was stirred 18 h at 25 ˚C. The reaction was then evaporated under vacuum to yield 19.6 g. The triphenylphosphine oxide was washed and filtered out with diethyl ether (2 x 25 mL), hexane (2 x 25 mL), and pentane (2 x 25 mL) evaporating under vacuum after each wash. The liquid was purified by gravity column, eluted with 40:1 hexane/ethyl acetate ($R_f$ 0.25) to yield 8.90 g. The final purification was by short path distillation; the product was distilled at 140-150 ˚C under reduced pressure to yield 3.5 g, 26% of a clear oil.

In a Parr flask the chloride, prepared above, (1.65 g, 6.25 mmol) was dissolved in ethanol (80 mL) followed by the addition of methylamine (40% aq., 5.38 mL, 62.5 mmol) and placed in a Parr shaker apparatus for 18 h at 90 ˚C. At the end of 18 h the solution was evaporated under vacuum. The crude product was the washed 0.1 M NaOH (3 x 25 mL), dried (anh. $Na_2SO_4$), filtered, and evaporated to 1.4 g. This material was further purified by column chromatography (10% methanol/$CH_2Cl_2$) to 0.566 g. The maleate salt was then made by adding a solution of maleic acid (0.234 g, 2.01 mmol) in ethyl acetate (5 mL) to the solution of amine in ethyl acetate (50 mL). To this solution was added hexane (40 mL). The precipitate was then filtered and dried to yield 0.430 g, 26.6% of product as its maleate.

## Synthesis of Compound 222

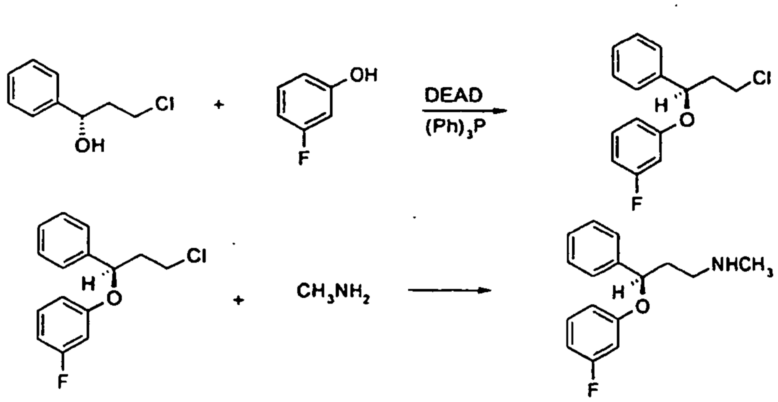


In a 500-mL round bottom flask, triphenylphosphine (19.93 g, 76.18 mmol) was dissolved in THF (50 mL). To this solution was added (*S*)-(+)-chloro-1-phenyl-1-propanol (10.00 g, 58.6 mmol) and 3-fluorophenol (6.57 g, 58.6 mmol). The reaction was stirred in an ice bath for 10 min. Finally diethyl azodicarboxylate (13.27 g, 76.18 mmol) was added

dropwise over 5 min. The reaction was stirred 18 h at 25 ˚C. The reaction was then evaporated under vacuum to yield 17.5 g. The supernatant was decanted and the triphenylphosphine oxide residue was washed with diethyl ether (2 x 25 mL), hexane (2 x 25 mL), and pentane (2 x 25 mL). The combined washings were evaporated and the residue was purified by gravity column, eluted with 40:1 hexane/ethyl acetate ($R_f$ 0.25) to yield 4.5 g, 29% of product.

In a Parr flask the chloride prepared above (1.65 g, 6.25 mmol) was dissolved in ethanol (80 mL) followed by the addition of methylamine (40% aq., 5.38 mL, 62.5 mmol) and placed in a Parr automatic apparatus for 18 h at 90˚C. At the end of 18 h the solution was evaporated under vacuum. The residue was then washed with 0.1 M NaOH (3 x 25 mL), dried (anh. $Na_2SO_4$), filtered, and evaporated to provide 1.25 g. The maleate salt was prepared by adding maleic acid (0.522 g, 4.50 mmol) in ethyl acetate (5 mL) to a solution of amine in ethyl acetate (50 mL). To this solution was added hexane (40 mL) to precipitate the product, which was collected and dried to provide 0.845 g, 52.2% overall.

Synthesis of Compound 185

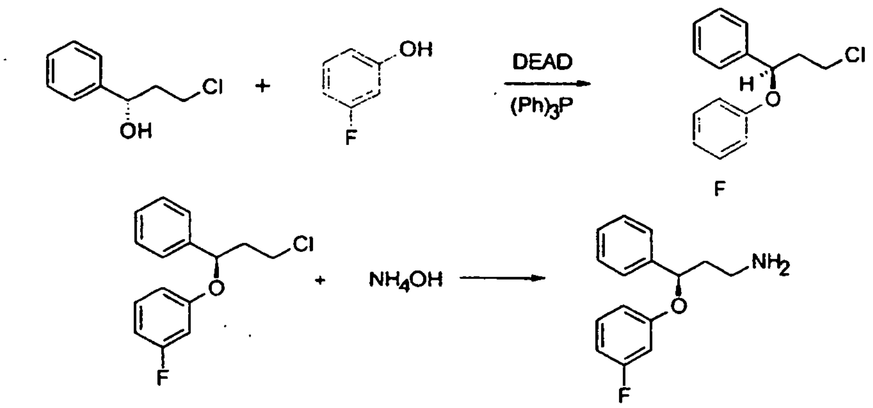


In a 500-mL round bottom flask, triphenylphosphine (19.93 g, 76.18 mmol) was dissolved in THF (50 mL). To this solution was added (*S*)-(+)-chloro-1-phenyl-1-propanol (10.00 g, 58.6 mmol) and 3-fluorophenol (6.57 g, 58.6 mmol). Stirred in an ice bath for 10 min. Finally, diethyl azodicarboxylate (13.27 g, 76.18 mmol) was added dropwise over 5 min. The reaction was stirred 18 h at 25 ˚C. The reaction was then evaporated under vacuum to yield 17.5 g. The triphenylphosphine oxide was washed and filtered out with ether (2 x 25 mL), hexane (2 x 25 mL), and pentane (2 x 25 mL) evaporating under vacuum after each wash. The liquid was purified by gravity column, eluted with 40:1 hexane/ethyl acetate ($R_f$ 0.25) to yield 4.5 g, 29% of product.

In a Parr flask the chloride prepared above (1.2 g, 4.54 mmol) was dissolved in ethanol (50 mL). Ammonium hydroxide (40 mL) was added and the reaction mixture was placed in an automatic Parr apparatus for 18 h at 90 ˚C. At the end of 18 h the solution was evaporated under vacuum. The oil was then transferred to a separatory funnel and washed with 0.1 M NaOH (2 x 25 mL) and water (2 x 25 mL), dried $Na_2SO_4$, filtered, and evaporated under vacuum. To a solution of resulting amine (0.703 g) in ethyl acetate (25 mL) was added a solution of maleic acid (0.309 g, 2.66 mmol) in ethyl acetate (5 mL). Hexane (10 mL) was added and the solution stirred until a solid was formed. The resulting white solid was filtered and dried in a vacuum oven to yield 0.600 g of product.

Synthesis of Compound 235

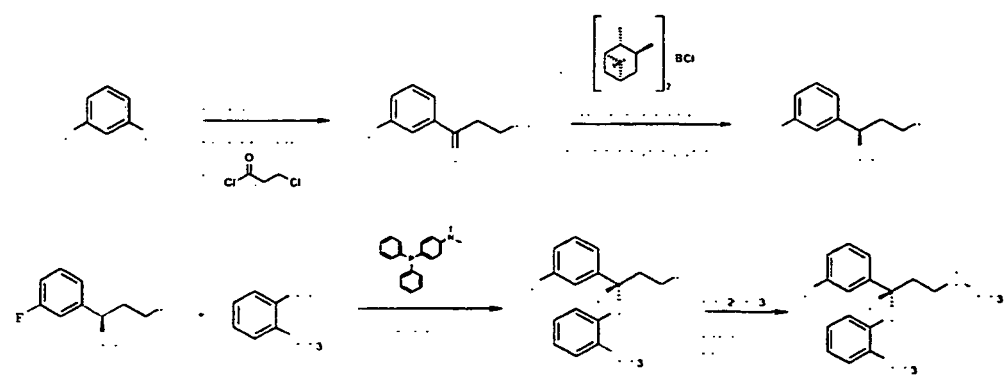

Compound 235 was synthesized in a four-step reaction sequence starting from commercially available materials. The Grignard reagent from 3-bromofluorobenzene was reacted with chloropropionyl chloride in the presence of copper bromide and lithium bromide to provide the chlorofluoropropiophenone. Following a method reported in the literature by Srebnik, M., Ramachandran, P.V., and Brown, H.C. (J. Org. Chem., 1988, 53, 2916-2920), the carbonyl group was stereoselectively reduced using (+)-*B*-chlorodiisoinocampheylborane. The resulting enantiomeric alcohol was then converted with stereochemical inversion to its phenolic ether. The chloride functionality was then reacted with methylamine to provide the final product.

3'-Chloro-3-fluoropropiophenone. The Grignard reagent was prepared as follows. Under nitrogen, in an oven-dried, 2000-mL, 3-necked flask, to a suspension of magnesium turnings [Alpha Aesar , prewashed with diethyl ether (3 x 100 mL), 6.08 g, 250 mmol] in dry diethyl ether (400 mL) was added one crystal of iodine. A solution of 3-bromofluorobenzene (27.9 mL, 250 mmol) in diethyl ether (30 mL) was added dropwise. The reaction mixture was heated to reflux on a hotplate. After initiation, the heat source was removed and the remainder of the bromide was added dropwise over a period of 40 min at such a rate as to maintain steady reflux. The reaction mixture was then heated to reflux for an additional 30 min. The Grignard reaction was then cooled to room temperature with a water bath. The reaction mixture was then cooled in an ice bath and lithium bromide (43.4 g, 500 mmol), copper (I) bromide (35.9 g, 250 mmol), and diethyl ether (300 mL) were added. The ice bath was removed and the dark reaction mixture was allowed to stir at 25 ˚C for 30 min.

A solution of 3-chloropropionyl chloride (23.9 mL, 250 mmol) in diethyl ether (300 mL) was cooled in an ice bath. The organocuprate solution was then transferred via cannula, over a period of 25 min, to the stirring acid chloride. The dark residue remaining in the flask was washed with diethyl ether (2 x 50 mL) and the washings added to the solution of acid chloride. The ice bath was removed and the reaction mixture was then allowed to stir at 25 ˚C.

The reaction mixture was then cooled in an ice bath and satd. aq. $NH_4Cl$ (500 mL) was added to the 0 ˚C reaction mixture with stirring until the color changed from black to green. The mixture was then separated and the aqueous layer was back-extracted with diethyl ether (3 x 150 mL). The combined organic layers were washed with satd. aq. $NH_4Cl$ (100 mL), dried (anh. $Na_2SO_4$, $Mg_2SO_4$), and evaporated under vacuum to provide 34.1 g, 73.0% of an oil. The crude material was dissolved in refluxing hexane (175 mL), filtered through paper, and was chilled 2 h in an ice bath. The resulting low-melting powder was dried to provide 21.3 g, 45.6% of purified ketone.

3-Chloro-3'-fluorophenylpropan-1-ol. Under argon, to a -25 ˚C (dry ice/acetonitrile/water bath) solution of (+)-DIP-Chloride$^{tm}$ [((+)-*B*-chlorodiisopinocampheylborane), 16.4 g, 51.0 mmol] in THF (35 mL) was added the ketone (8.65 g, 46.4 mmol). The reaction mixture was then cooled to -25 ˚C and then allowed to slowly warm to room temperature while stirring for 7 h. The solution was then evaporated under vacuum and dried under high vacuum (0.1 mm, 50 ˚C) for 18 h. The residue was then dissolved in diethyl ether (200 mL). Diethanolamine (13.4 mL 140 mmol) was added and the reaction mixture was stirred for 2 h. The resulting mixture was then filtered and the residue was washed with pentane (3 x 25 mL). The combined filtrate and washings were evaporated in vacuo to provide 16.7 g of an oil which was then placed under high vacuum (0.23 mm, 60˚C) for 18 h to provide 9.51 g. The material was chromatographed on silica gel [elution with hex/EtOAc (10:1)] to provide 5.11 g, 58.4% of an oil.

(S)-1-[3-Chloro-1-(3-fluorophenyl)propoxy]-2-methylbenzene. To a solution of 4-(dimethylamino)phenyldiphenylphosphine (1.98 g, 6.49 mmol) in THF (40 mL) was added 3′-chloro-3-flourophenylpropanol .(1.02 g, 5.41 mmol) followed by ortho-cresol (0.760 g, 7.03 mmol). The reaction mixture was then placed in an ice bath and stirred for 10 min. Diisopropyl azodicarboxylate (DIAD, 1.28 mL 6.49 mmol) was then added dropwise over a period of 1 min. The ice bath was then removed and the reaction mixture was stirred at room temperature for 4 h. The reaction mixture was then poured into diethyl ether (100 mL) and was washed with 1 M NaOH (2 x 25 mL), 1 M HCl (2 x 25 mL), and brine (25

mL). The organic layer was dried (anh. $Na_2SO_4$) and evaporated to provide 2.82 g of an oil which crystallized on standing. This material was chromatographed [hex/EtOAc (20:1)] to provide 0.87 g (57.6%) of the product as an oil.

(S)-*N*-Methyl-3-(3-fluorophenyl)-3-[(2-methylphenyl)oxy]propylamine (Compound 235). In a 500-mL Parr flask (Parr Apparatus), to a solution of the chloro ether (0.435 g, 1.56 mmol) in ethanol (10 mL) was added methylamine (40% aq., 20 mL). The reaction was sealed, heated to 80 ˚C, and shaken for 24 h. The reaction mixture was then cooled to room temperature and the volatiles were evaporated under vacuum. The resulting oil was dissolved in diethyl ether and the organic layer was washed with satd. aq. NaCl (2 x 10 mL), dried (anh. $Na_2SO_4$), and rotary evaporated to provide the crude free base as an oil.

This material was purified by reversed-phase HPLC (20-100% acetonitrile in 0.1% aq. HCl gradient) and lyophilized. The amine HCl salt was dissolved in $CHCl_3$ (50 mL) and " free based" with satd. aq. $NaHCO_3$ (10 mL). The resulting oil was dissolved in EtOAc (5 mL) and a solution of maleic acid (91 mg) in EtOAc (5 mL) was added. The solution was evaporated to an oil that slowly crystallized to provide 275 mg of the final product.

Synthesis of Compound 232

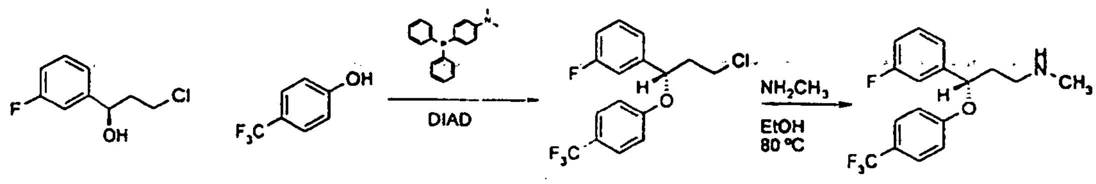


compound 232

Compound 232 was synthesized in a four-step reaction sequence starting from commercially available materials. In a similar pathway as compound 235 was prepared, the Grignard reagent from 3-bromofluorobenzene was reacted with chloropropionyl chloride in the presence of copper bromide and lithium bromide to provide the chlorofluoropropiophenone. Following a method reported in the literature by Srebnik, M., Ramachandran, P.V., and Brown, H.C. (J. Org. Chem. 1988, 53, 2916-2920), the carbonyl group was stereoselectively reduced using (+)-*B*-chlorodiisopropylcampheylborane. The resulting enantiomeric alcohol was then converted with stereochemical inversion to its phenolic ether. The chloride functionality was then reacted with methylamine to provide the final product.

(S)-1-(3-Chloro-1-(3-fluorophenyl)propoxy)-4-trifluoromethylbenzene. To a solution of 4-(dimethylamino)phenyldiphenylphosphine (1.98 g, 6.49 mmol) in THF (40 mL) was added 3'-chloro-3-flourophenylpropanol (1.02 g, 5.41 mmol) followed by para-trifluoromethylphenol (1.14 g, 7.03 mmol). The reaction mixture was then placed in an ice bath and stirred for 10 min. Then, diisopropyl azodicarboxylate (DIAD, 1.28 mL 6.49 mmol) was added dropwise over a period of 1 min. The ice bath was then removed and the reaction mixture was stirred at room temperature for 4 h. The reaction mixture was then poured into diethyl ether (100 mL) and washed with 1 M NaOH (2 x 25 mL), 1 M HCl (2 x 25 mL), and brine (25 mL). The organic layer was dried (anh. $Na_2SO_4$) and evaporated to provide 2.82 g of an oil which crystallized on standing. This material was chromatographed (hex/EtOAc [20:1]) to provide 1.06 g (58.8%) of product as an oil.

(S)-*N*-Methyl-3-(3-fluorophenyl)-3-[(4-trifluoromethylphenyl)oxy]propylamine (Compound 232). In a 500-mL Parr flask (Parr Apparatus), to a solution of the chloro ether (0.503 g, 1.51 mmol) in ethanol (10 mL) was added aqueous methylamine (40% aq., 20 mL). The reaction was sealed, was heated to 80 ˚C (40 psig), and was shaken for 18 h. The reaction mixture was then cooled to room temperature and the volatiles were evaporated under vacuum. The resulting oil was dissolved in diethyl ether (100 mL), the organic layer washed with satd. aq. NaCl (2 x 10 mL), dried (anh. $Na_2SO_4$), and rotary evaporated to provide the crude free base as an oil. This material was chromatographed on silica gel (0-10% MeOH/$CHCl_3$, gradient elution) to provide 450 mg of product as an oil.

This material was further purified by reversed-phase HPLC (20%-100% acetonitrile in 0.1% aq. HCl, gradient) and lyophilized. The amine HCl salt was dissolved in $CHCl_3$ (50 mL) and free based with satd. aq. $NaHCO_3$ (10 mL). The resulting oil (230 mg) was dissolved in EtOAc (5 mL) and a solution of maleic acid (81 mg) in EtOAc (5 mL) was added. The solution was evaporated under high vacuum to an oil that slowly crystallized to provide 150 mg of final product.

Synthesis of Compound 225

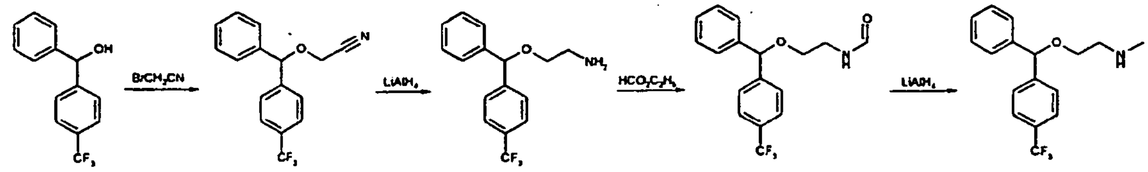


Compound 225 was synthesized in four steps from commercially available starting materials. 4-Trifluoromethylbenzhydrol was reacted with bromoacetonitrile under basic phase-transfer conditions to synthesize the benzhydrylacetonitrile ether. The nitrile was then reduced to the primary amine. The amine was *N*-methylated in two steps by reduction of its corresponding formamide.

[1-Phenyl-1-(*p*-trifluoromethylphenyl)methoxy]acetonitrile. A mixture of 4-trifluoromethylbenzhydrol (24.36 g, 97 mmol), tetrabutylammonium hydrogen sulfate (0.45 g, 1.3 mmol), $CH_2Cl_2$ (40 mL), and 50% aq. NaOH (10.53 g NaOH, 263 mmol) was stirred at 20 ˚C for 1 h. The reaction mixture was then cooled to 0 ˚C and bromoacetonitrile (12.5 mL, 179 mmol) was added dropwise. The reaction mixture was stirred at 0 ˚C for 3 h. The reaction mixture was diluted with diethyl ether (200 mL) and washed with water until the washings were neutral (10 x 50 mL). The organic layer was dried (anh. $MgSO_4$), filtered, and rotary evaporated (33.73 g, 120%). This material was chromatographed ($CHCl_3$) through silica gel to yield 21.0 g (75%) of the product as an oil.

[1-Phenyl-1-(*p*-trifluoromethylphenyl) methoxy] ethylamine. To lithium aluminum hydride (1.10 g, 29.0 mmol) was added anh. diethyl ether (100 mL). This mixture was stirred under argon flow for 5 min. The nitrile prepared above (5.00 g, 17.2 mmol) in anh. diethyl ether (5 mL) was added over a period of 2 min (rinsing the nitrile container with 5 mL diethyl ether). The reaction was stirred for 30 min [TLC (4:1 hex/EtOAc) showed no starting material after 15 min]. To the reaction mixture was slowly added ethyl acetate (5 mL), followed by water (1.1 mL), 5 M NaOH (1.1 mL), and then more water (3.3 mL). The reaction mixture was filtered through paper, and the filtrate was dried (anh. $Na_2SO_4$) and rotary evaporated (75 ˚C) to provide 4.25 g (83.8%) of the product as an oil. This oil was flash chromatographed ($CHCl_3$, 1:20 MeOH/$CHCl_3$, step gradient) through flash silica gel to provide 3.52 g (69.4%) of the product as an oil.

[1-Phenyl-1-(*p*-trifluoromethylphenyl)methoxy]ethylamine formamide. The primary amine prepared above (2.13 g, 7.21 mmol) was dissolved in ethyl formate (40 mL, 500 mmol). The reaction solution was refluxed for 15 h, and was then rotary evaporated. This provided 2.42 g (104%) of the product as an oil. This oil was flash chromatographed (hexanes, 1:1 hex/EtOAc, EtOAc, step gradient) through flash silica gel to provide 1.71 g (73.3%) of the product as an oil.

*N*-Methyl-[1-phenyl-1-(*p*-trifluoromethylphenyl)methoxy]ethylamine. To lithium aluminum hydride (0.30 g, 7.9 mmol) was added anh. diethyl ether (20 mL). This mixture was stirred under argon flow for 5 min. The formamide prepared above (1.68 g, 5.20 mmol) in anh. diethyl ether (5 mL) was added over a period of 1 min (rinsing the nitrile container with 5 mL diethyl ether). The reaction was stirred for 20.5 h [TLC (EtOAc) showed almost no starting material after 4.5 h]. To the reaction mixture was slowly added EtOAc (0.3 mL), followed by water (0.3 mL), 5 M NaOH (0.3 mL), and then more water (0.9 mL). The reaction mixture was filtered through paper, and the filtrate was dried (anh. $Na_2SO_4$) and rotary evaporated (75 ˚C) to provide 1.43 g (89.0%) of the product as an oil. This oil was flash chromatographed (EtOAc, 1:20 MeOH/$CHCl_3$, 1:1 MeOH/$CHCl_3$, step gradient) through flash silica gel to provide 0.91 g (57%) of the product as an oil. This material was dissolved in EtOAc (2 mL) and filtered. To the filtrate was added a solution of maleic acid (0.31 g, 2.7 mmol) in EtOAc (5 mL). To this solution was added diethyl ether (15 mL). Crystals formed, were filtered, washed with diethyl ether (2 x 10 mL), and dried under high vacuum to provide 888 mg (40.2%) of the maleate salt as a finely crystalline solid.

## Claims

1. Use of a compound having the following chemical structure:

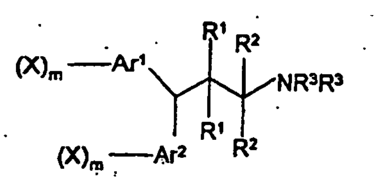

wherein each X is independently selected from the group consisting of -F, -Cl, $-OCF_3$, or $-CF_3$;
$Ar^1$ and $Ar^2$ are each independently selected from the group consisting of phenyl, naphthyl, thiofuranyl, tetrahydrohaphthyl, furanyl, tetrahydrofuranyl, pyridyl, quinolinyl, isoquinolinyl, tetrahydroquinolinyl, tetrahydroisoquinolinyl, cyclohexyl, cycloheptyl, and cyclopentyl;
each R is -H;
each $R^2$ is -H ;
one. $R^3$ is -H, and the other $R^3$ is either -H or $-CH_3$;
each m is 1;
provided that if one $R^3$ is -H and the other $R^3$ is $-CH_3$ then both $X_m$'s are not 4-Cl;
or a pharmaceutically acceptable salt thereof;
for the preparation of a medicament for treating a patient for depression.

**2.** Use of a compound having the chemical structure:

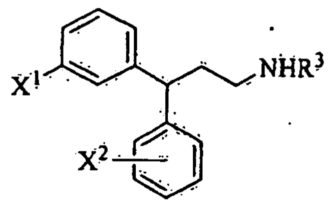

wherein $X^1$ is either -Br, -Cl, -F, -I, $-CF_3$, alkyl, -OH, $-OCF_3$, -O-alkyl, or -O-acyl;
$X^2$ is either -Br, -Cl, -F, -I, $-CF_3$, alkyl, -OH, $-OCF_3$, -O-alkyl, or -O-acyl; and
$R^3$ is either -H or $-CH_3$;
or a pharmaceutically acceptable salt thereof;
for the preparation of a medicament for treating a patient for depression.

**3.** Use of claim 2, wherein $X^1$ is -F, -Cl, $-OCF_3$ or $-CF_3$; and $X^2$ is either $2\text{-}OCH_3$, $2\text{-}CH_3$, 3-F, $3\text{-}CF_3$, or $4\text{-}CF_3$.

**Patentansprüche**

**1.** Verwendung einer Verbindung der folgenden chemischen Struktur:

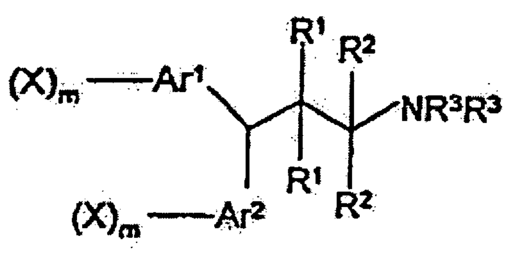

wobei jedes X unabhängig voneinander aus -F, -Cl, $-OCF_3$ oder $-CF_3$ ausgewählt ist;
$Ar^1$ und $Ar^2$ jeweils unabhängig voneinander aus Phenyl, Naphthyl, Thiofuranyl, Tetrahydronaphthyl, Furanyl, Tetrahydrofuranyl, Pyridyl, Chinolinyl, Isochinolinyl, Tetrahydrochinolinyl, Tetrahydroisochinolinyl, Cyclohexyl, Cycloheptyl und Cyclopentyl ausgewählt sind;
jeder Rest $R^1$ -H ist;
jeder Rest $R^2$ -H ist;
ein Rest $R^3$ -H ist und der andere Rest $R^3$ entweder -H oder $-CH_3$ ist;
jedes m gleich 1 ist;
mit der Maßgabe, dass, wenn ein Rest $R^3$ -H ist und der andere Rest $R^3$ $-CH_3$ ist, dann beide $X_m$ nicht 4-Cl sind;
oder eines pharmazeutisch verträglichen Salzes davon;
zur Herstellung eines Medikaments zur Behandlung von Depression bei einem Patienten.

**2.** Verwendung einer Verbindung der chemischen Struktur:

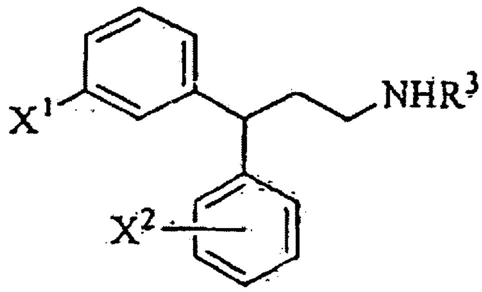

wobei $X^1$ entweder -Br, -Cl, -F, -I, $-CF_3$, Alkyl, -OH, $-OCF_3$, -O-Alkyl oder -O-Acyl ist;
$X^2$ entweder -Br, -Cl, -F, -I, $-CF_3$, Alkyl, -OH, $-OCF_3$, -O-Alkyl oder -O-Acyl ist; und
$R^3$ entweder -H oder $-CH_3$ ist;
oder eines pharmazeutisch verträglichen Salzes davon;
zur Herstellung eines Medikaments zur Behandlung von Depression bei einem Patienten.

**3.** Verwendung nach Anspruch 2, wobei $X^1$ -F, -Cl, $-OCF_3$ oder $-CF_3$ ist; und $X^2$ entweder $2\text{-}OCH_3$, $2\text{-}CH_3$, 3-F, $3\text{-}CF_3$ oder $4\text{-}CF_3$ ist.

## Revendications

**1.** Utilisation d'un composé ayant la structure chimique suivante:

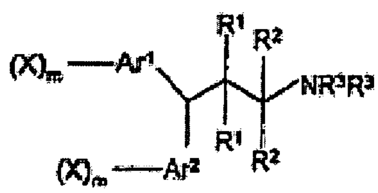

dans laquelle chaque X est indépendamment choisi dans le groupe se composant de -F, -Cl, $-OCF_3$ ou $-CF_3$;
$Ar^1$ et $Ar^2$ sont chacun indépendamment choisis dans le groupe se composant du phényle, du naphthyle, du thiofuranyle, du tétrahydronaphthyle, du furanyle, du tétrahydrofuranyle, du pyridyle, du quinolinyle, de l'isoquinolinyle, du tétrahydroquinolinyle, du tétrahydroisoquinolinyle, du cyclohexyle, du cycloheptyle, et du cyclopentyle ;
chaque $R^1$ est -H ;
chaque $R^2$ est -H ;
un $R^3$ est -H ; et l' autre $R^3$ est soit -H soit $-CH_3$ ;
chaque m est 1 ;
à condition que si un $R^3$ est -H et l'autre $R^3$ est $-CH_3$, alors les deux $X_m$ ne sont pas 4-Cl ;
ou un de leurs sels pharmaceutiquement acceptables ;
pour la préparation d'un médicament destiné au traitement d'un patient souffrant de dépression.

**2.** Utilisation d'un composé ayant la structure chimique :

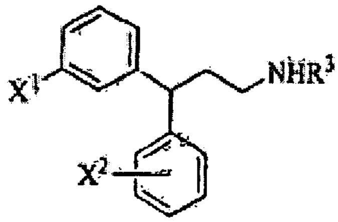

dans laquelle $X^1$ est soit -Br, -Cl, -F, -I, $-CF_3$, un alkyle, -OH, $-OCF_3$, -O-alkyle, ou -O-acyle;
$x^2$ est soit -Br, -Cl, -F, -I, $-CF_3$, un alkyle, -OH, $-OCF_3$, -O-alkyle, ou -O-acyle ; et
$R^3$ est soit -H soit $-CH_3$;
ou un de leurs sels pharmaceutiquement acceptables ;
pour la préparation d'un médicament destiné au traitement d'un patient souffrant de dépression.

**3.** Utilisation selon la revendication 2, dans laquelle $X^1$ est -F, -Cl, $-OCF_3$, ou $-CF_3$ ; et $X^2$ est soit 2-$OCH_3$, 2-$CH_3$, 3-F, 3-$CF_3$, ou 4-$CF_3$.